# EUROPEAN PATENT APPLICATION

(11) **EP 4 567 039 A1**
(43) Date of publication of application: **11.06.2025**
(21) Application number: 23862198.1
(22) Date of filing: 25.08.2023
(51) Int. Cl.: C07D 491/22, A61P 35/00, A61K 31/4745, A61K 31/496, A61K 47/68

(54) **CAMPTOTHECIN-7-ETHYLAMINE DERIVATIVE, PREPARATION METHOD THEREFOR, AND USE THEREOF**

(30) Priority: 09.09.2022 CN 202211104696
(71) Applicant: Hangzhou Adcoris Biopharma Co., Ltd, Hangzhou, Zhejiang 310056 (CN)
(72) Inventor: HUANG, Yunsheng, Hangzhou Xianghu Biotechnology Innovation Center, No. 1688, Tianma Road, Wenyan Street, Xiaoshan District Hangzhou, Zhejiang 311258 (CN); ZHANG, Shiyi, Hangzhou Xianghu Biotechnology Innovation Center, No. 1688, Tianma Road, Wenyan Street, Xiaoshan District Hangzhou, Zhejiang 311258 (CN); HUI, Shun, Hangzhou Xianghu Biotechnology Innovation Center, No. 1688, Tianma Road, Wenyan Street, Xiaoshan District Hangzhou, Zhejiang 311258 (CN)
(74) Representative: Elzaburu S.L.P.
(86) International application number: PCT/CN2023/114928
(87) International publication number: WO 2024/051502

(57) **Abstract**

The present invention provides a camptothecin-7-ethylamine derivative or a pharmaceutically acceptable salt thereof, a stereoisomer thereof or a prodrug thereof, a preparation method therefor, and use thereof. The camptothecin-7-ethylamine derivative has a structure represented by formula (1).

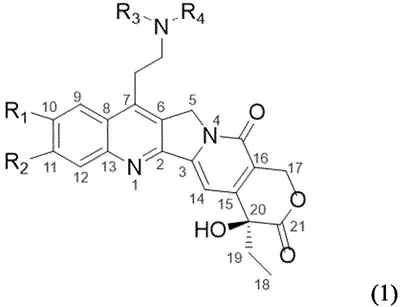

## Description

### TECHNICAL FIELD

The present disclosure relates to the field of medicine. Specifically, the present disclosure provides the preparation of a series of camptothecin-7-ethylamino derivatives and the use thereof in the treatment of tumors.

### BACKGROUND

Camptothecin is a natural product extracted from the Chinese plant *Camptotheca acuminata* and has inhibitory activity against topoisomerase Top1, especially showing strong inhibitory activity against the complex formed by Top1-DNA. Camptothecin is a broad-spectrum antitumor drug that has significant therapeutic effects on gastric cancer, esophageal cancer, lung cancer and bladder cancer, etc. Wherein Irinotecan and Topotecan have been approved for the treatment of various cancers in many countries. Another camptothecin derivative, Belotecan, has been approved for the treatment of SCLC (small cell lung cancer) and ovarian cancer in Republic of Korea. The main drawbacks of camptothecin-based antitumor drugs are their toxicity, poor solubility, and the development of drug resistance by tumor cells.

The present disclosure provides a series of camptothecin-7-ethylamine derivatives, characterized by having improved solubility and enhanced antitumor activity, and having potential application value in the treatment of tumors.

### SUMMARY

The present disclosure provides a series of camptothecin-7-ethylamine derivatives or pharmaceutically acceptable salts, stereoisomers or prodrugs thereof, as well as preparation methods and uses thereof in the anti-tumor field.

In one aspect of the present disclosure, it provides a compound represented by Formula 1 or a pharmaceutically acceptable salt, a stereoisomer, or a prodrug thereof, 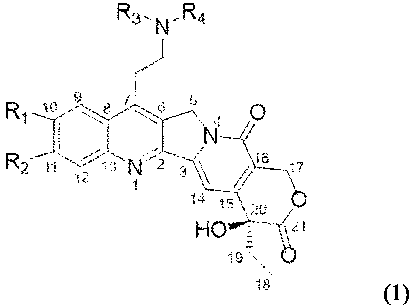
wherein, each of R₁ and R₂ independently represents halogen, hydroxy, alkyl, alkoxy, or R₁ and R₂collectively form a methylenedioxy bridge or an ethylenedioxy bridge;
each of R₃ and R₄ independently represents hydrogen, hydroxy, alkyl, alkoxy, cycloalkyl, heterocycloalkyl, aryl, heteroaryl, acyl, sulfonyl, or R₃, R₄ and the nitrogen atom to which they connect collectively form a heterocycloalkyl or heteroaryl, the alkyl, alkoxy, cycloalkyl, alkylacyl, sulfonyl, heterocycloalkyl, aryl, or heteroaryl is optionally substituted by R,
R is selected from halogen, hydroxy, alkyl, alkoxy, cycloalkyl, azide or 5 to 7 membered heteroaryl.

In one embodiment, each of R₁ and R₂ independently represents halogen, hydroxy, C₁-C₆ alkyl, C₁-C₆ alkoxy, or R₁ and R₂ collectively form a methylenedioxy bridge or an ethylenedioxy bridge.

In one embodiment, each of R₁ and R₂ independently represents halogen, methyl.

In one embodiment, each of R₁ and R₂ independently represents methyl, F, Cl, Br, I.

In one embodiment, each of R₁ and R₂ independently represents methyl, F.

In one embodiment, R₁ and R₂ collectively form a methylenedioxy bridge or an ethylenedioxy bridge.

In one embodiment, each of R₃ and R₄ independently represents hydrogen, C₁-C₆ alkyl, C₁-C₆ alkoxy, C₃-C₆ cycloalkyl, 4 to 8 membered heterocycloalkyl, 5 to 12 membered aryl, 5 to 12 membered heteroaryl, C₁-C₆ alkyl-acyl, C₁-C₆ alkyl-sulfonyl, C₃-C₆cycloalkyl-sulfonyl, or R₃, R₄ and the nitrogen atom to which they connect collectively form a 4 to 8 membered heterocycloalkyl, the C₁-C₆ alkyl, C₁-C₆ alkoxy, C₃-C₆ cycloalkyl, 4 to 8 membered heterocycloalkyl, 5 to 12 membered aryl, 5 to 12 membered heteroaryl, C₁-C₆ alkyl-acyl, C₁-C₆ alkyl-sulfonyl, or C₃-C₆ cycloalkyl-sulfonyl is optionally substituted by R.

In one embodiment, each of R₃ and R₄ independently represents C₁-C₆ alkyl, C₃-C₆ cycloalkyl, 5 to 6 membered heterocycloalkyl, 5 to 6 membered heteroaryl, C₁-C₃ alkyl-acyl, C₁-C₃ alkyl-sulfonyl, C₃-C₆ cycloalkyl-sulfonyl, or R₃, R₄ and the nitrogen atom to which they connect collectively form a 5 to 6 membered heterocycloalkyl, the C₁-C₆ alkyl, C₃-C₆ cycloalkyl, 5 to 6 membered heterocycloalkyl, 5 to 6 membered heteroaryl, C₁-C₃ alkyl-acyl, C₁-C₃ alkyl-sulfonyl, or C₃-C₆ cycloalkyl-sulfonyl is optionally substituted by R.

In one embodiment, each of R₃ and R₄ independently represents C₁-C₆ alkyl, C₃-C₆cycloalkyl, 5 to 6 membered heterocycloalkyl, 5 to 6 membered heteroaryl, C₁-C₃ alkyl-acyl, C₁-C₃ alkyl-sulfonyl, C₃-C₆ cycloalkyl-sulfonyl, or R₃, R₄ and the nitrogen atom to which they connect collectively form a 5 to 6 membered heterocycloalkyl, the C₁-C₆ alkyl, C₃-C₆ cycloalkyl, 5 to 6 membered heterocycloalkyl, 5 to 6 membered heteroaryl, C₁-C₃ alkyl-acyl, C₁-C₃ alkyl-sulfonyl, or C₃-C₆ cycloalkyl-sulfonyl is optionally substituted by R, or R₃, R₄ and the nitrogen atom to which they connect collectively form a 5 to 6 membered heterocycloalkyl, the heterocycloalkyl, or heteroaryl contains 1-3 heteroatoms independently selected from N, O.

In one embodiment, R is selected from halogen, hydroxy, C₁-C₆ alkyl, C₁-C₆ alkoxy, C₃-C₆ cycloalkyl or azide.

In one embodiment, R is selected from F, Cl, Br, I, hydroxy, methyl, methoxy, cyclopropyl or azide.

In one embodiment, R is selected from F, hydroxy, methyl, methoxy, cyclopropyl or azide.

In one embodiment, R₃ and R₄ are each independently selected from the following groups:

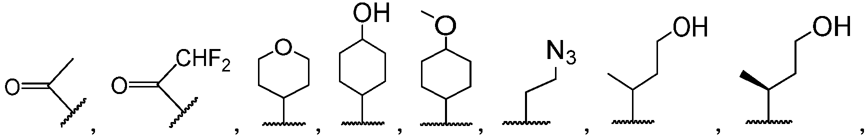

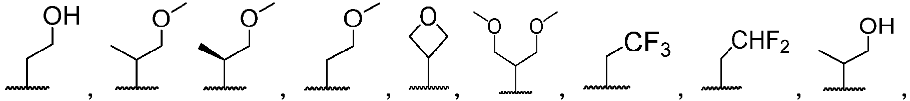

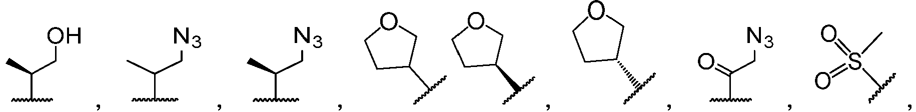

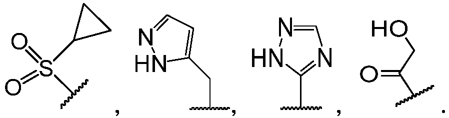

In one embodiment, R₃, R₄ and the nitrogen atom to which they connect collectively form pyrrolidinyl, piperidinyl, piperazinyl.

In one embodiment, R₃, R₄ and the nitrogen atom to which they connect collectively form

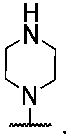

In one embodiment, R₁, R₂ are each independently or collectively the following structure: 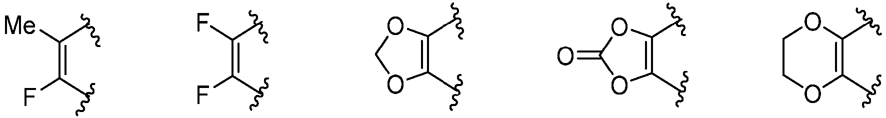 .

In one embodiment, the -NR₃R₄ is each independently or collectively the following structure:

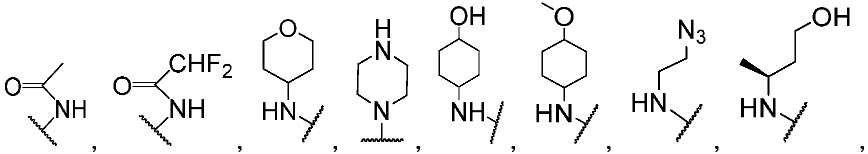

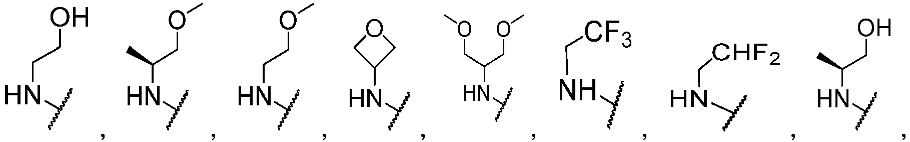

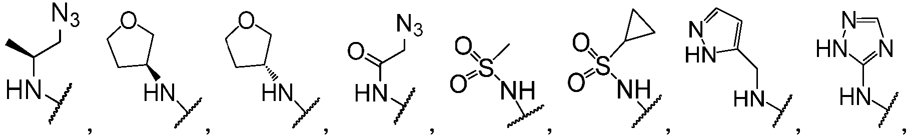

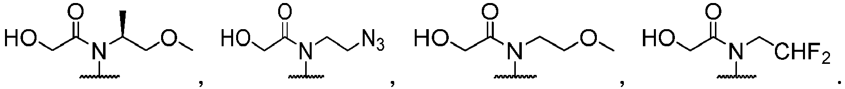

The present disclosure provides the following compounds or pharmaceutically acceptable salts, stereoisomers, or prodrugs thereof:

| | | |
|---|---|---|
| **1** | **2** | **3** |
| 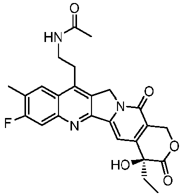 | 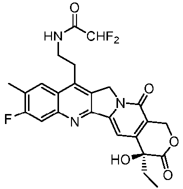 | 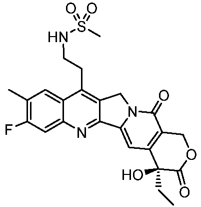 |
| **4** | **5** | **6** |
| 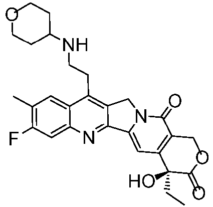 | 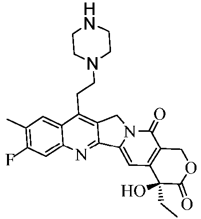 | 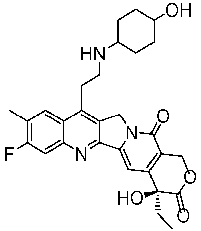 |
| **7** | **8** | **9** |
| 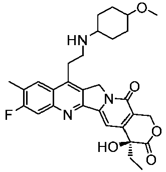 | 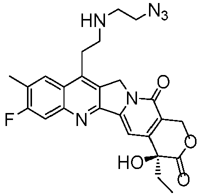 | 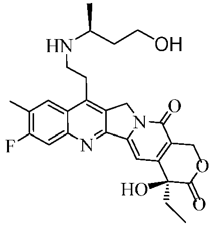 |
| **10** | **11** | **12** |
| 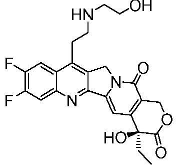 | 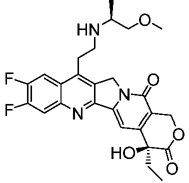 | 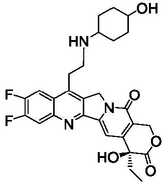 |
| **13** | **14** | **15** |
| 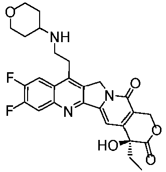 | 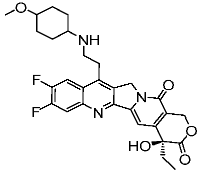 | 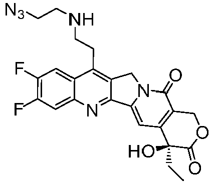 |
| **16** | **17** | **18** |
| 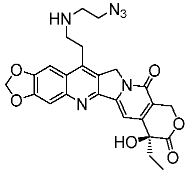 | 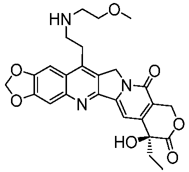 | 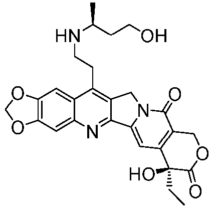 |
| **19** | **20** | **21** |
| 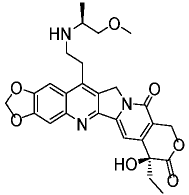 | 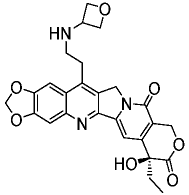 | 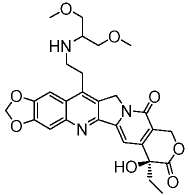 |
| **22** | **23** | **24** |
| 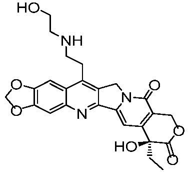 | 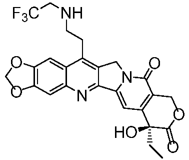 | 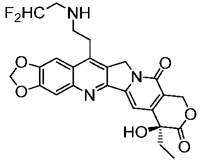 |
| **25** | **26** | **27** |
| 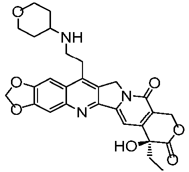 | 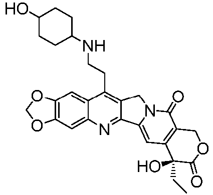 | 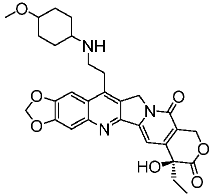 |
| **28** | **29** | **30** |
| 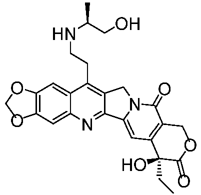 | 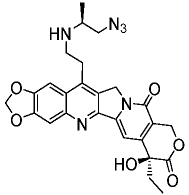 | 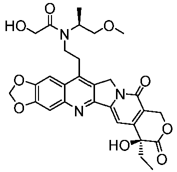 |
| **31** | **32** | **33** |
| 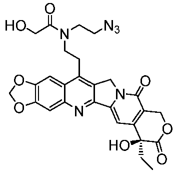 | 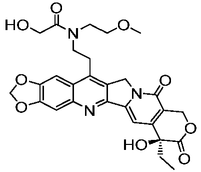 | 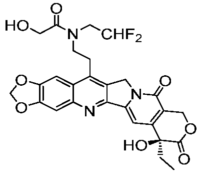 |
| **34** | **35** | **36** |
| 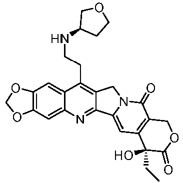 | 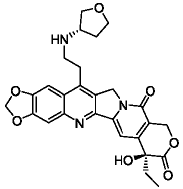 | 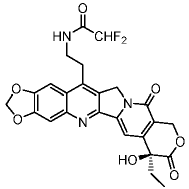 |
| **37** | **38** | **39** |
| 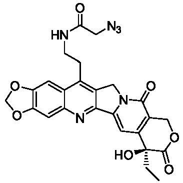 | 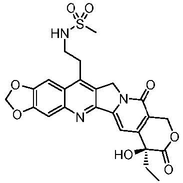 | 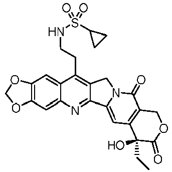 |
| **40** | **41** | **42** |
| 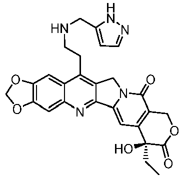 | 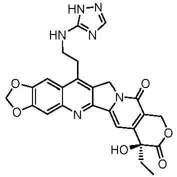 | 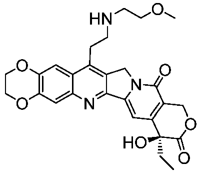 |
| **43** | **44** | **45** |
| 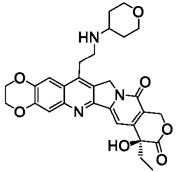 | 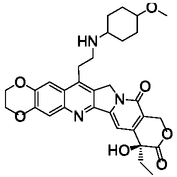 | 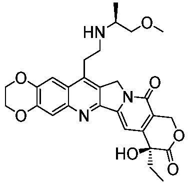 |

In another aspect of the present disclosure, it provides a method for preparing any one of the above compounds, the preparation method comprises steps selected from any one of the following synthesis routes:
synthesis route 1, wherein, it comprises the following processes:
   (1) 3,4-substituted aniline was reacted with the halopropionitrile to afford 3',4'-disubstituted-3-halo-6'-aminopropiophenone;
   (2) 3',4'-disubstituted-3-halo-6'-aminopropiophenone was subjected to a substitution reaction with an amine to afford 3',4'-disubstituted-3-alkylamino-6'-aminopropiophenone;
   (3) 3',4'-disubstituted-3-alkylamino-6'-aminopropiophenone was condensed with (S)-4-ethyl-4-hydroxy-7,8-dihydro-1H-pyranone-[3,4-f]indolizine-3,6,10(4H)-trione to afford the camptothecin-7-ethylamine derivatives.
Synthesis route 2, wherein, it comprises the following processes:
   (1) 3',4'-disubstituted-3-halopropiophenone was reacted with nitric acid to afford 6'-nitro-3',4'-disubstituted-3-halopropiophenone;
   (2) 6'-nitro-3',4'-disubstituted-3-halopropiophenone was subjected to a substitution reaction with an amine to afford 6'-nitro-3',4'-disubstituted-3-alkylamino-propiophenone;
   (3) 6'-nitro-3',4'-disubstituted-3-alkylamino-propiophenone was subjected to a reduction reaction to afford 6'-amino-3',4'-disubstituted-3-alkylamino-propiophenone;
   (4) 3',4'-disubstituted-3-alkylamino-6'-aminopropiophenone was condensed with (S)-4-ethyl-4-hydroxy-7,8-dihydro-1H-pyranone-[3,4-f]indolizine-3,6,10(4H)-trione to afford the camptothecin-7-ethylamine derivative.
Synthesis route 3, wherein, it comprises the following processes:
   (1) 3',4'-disubstituted acetophenone was nitrated to afford 6'-nitro-3',4'-disubstituted acetophenone;
   (2) 6'-nitro-3',4'-disubstituted acetophenone was condensed with formaldehyde and acidified to afford 6'-nitro-3',4'-disubstituted propenophenone;
   (3) 6'-nitro-3',4'-disubstituted propenophenone was subjected to a Michael addition reaction with an amine to afford 6'-nitro-3',4'-disubstituted propiophenone-3-amine;
   (4) 6'-nitro-3',4'-disubstituted propiophenone-3-amine was subjected to a reduction reaction to afford 6'-amino-3',4'-disubstituted propiophenone-3-amine;
   (5) 6'-amino-3',4'-disubstituted propiophenone-3-amine was subjected to a condensation reaction condensed with (S)-4-ethyl-4-hydroxy-7,8-dihydro-1H-pyranone-[3,4-f]indolizine-3,6,10(4H)-trione to afford the camptothecin-7-ethylamine derivatives.
Synthesis route 4, wherein, it comprises the following processes:
   (1) substituted 2-acetyl aniline was condensed with (S)-4-ethyl-4-hydroxy-7,8-dihydro-1H-pyranone-[3,4-flindolizine-3,6,10(4H)-trione to afford 7-methylcamptothecin;
   (2) 7-methylcamptothecin was subjected to a Mannich reaction with an amine in DMSO to afford the camptothecin-7-ethylamine derivative.

Preferably, the synthesis routes of the present disclosure are as follows:

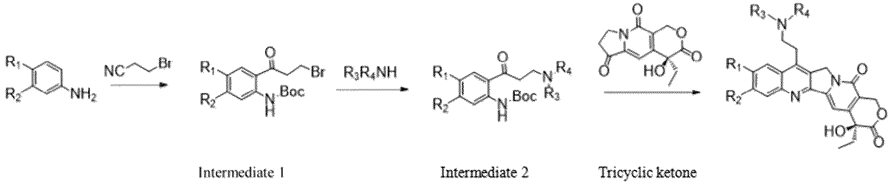

Synthesis route 1. Using 3,4-disubstituted aniline as the starting material, the target product is afforded through Friedel-Crafts acylation, nitration, amine substitution, reduction of the nitro group, and cyclization.

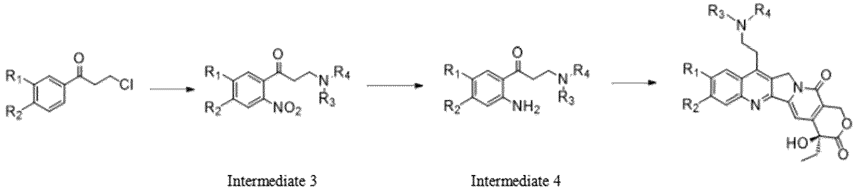

Synthesis route 2. Using 3,4-disubstituted 3'-chloropropiophenone as the starting material, the target product is afforded through amine substitution, nitration, reduction, and cyclization.

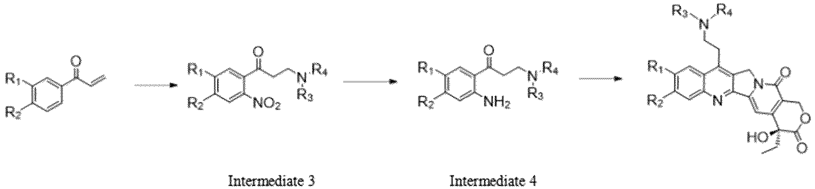

Synthesis route 3. Using 3,4-disubstituted propenophenone as the starting material, the target product is afforded through Michael addition of an amine, nitration, reduction, and cyclization.

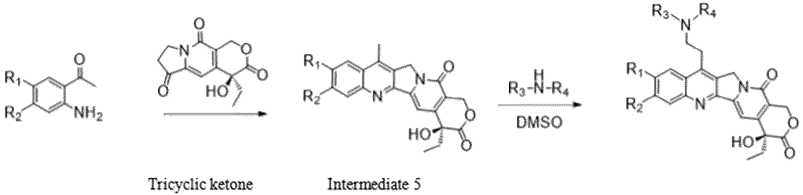

Synthesis route 4. 7-Methyl camptothecin (intermediate 5) was used as the substrate and was subjected to a Mannich reaction with an amine, DMSO to afford the 7-aminoethyl-substituted camptothecin product.

In one embodiment, the preparation method of the present disclosure comprises synthesis route 1 to synthesis route 4, including: 1) 3,4-substituted phenylamine was reacted with the corresponding halopropionitrile to afford the intermediate 3',4'-disubstituted-3-chloro-6'-aminopropiophenone (intermediate 1); 2) the intermediate 1 was subjected to a substitution reaction with a corresponding amine to afford 3',4'-disubstituted-3-alkylamino-6'-aminopropiophenone (intermediate 2); 3) the intermediate 2 was condensed with (S)-4-ethyl-4-hydroxy-7,8-dihydro-1H-pyranone-[3,4-flindolizine-3,6,10(4H)-trione (hereinafter referred to as "tricyclic ketone") to afford the corresponding product; 4) the intermediate 3 was afforded by reacting 3',4'-disubstituted-3-chloropropiophenone with various amines, substituted amines, protected amines, protected amino hydroxy, methoxy hydroxylamine, etc; 5) the intermediate 4 was afforded by nitration and reduction of intermediate 3; 6) the intermediate 4 was condensed with tricyclic ketone to afford the corresponding product; 7) 6'-nitro-3',4'-disubstituted propenophenone was subjected to a Michael addition reaction with an amine to afford 6'-nitro-3',4'-disubstituted propiophenone-3-amine (intermediate 3); 8) 6'-nitro-3',4'-disubstituted propiophenone-3-amine was reduced to afford 6'-amino-3',4'-disubstituted propiophenone-3-amine (intermediate 4); 9) 6'-amino-3',4'-disubstituted propiophenone-3-amine was subjected to a condensation reaction with "tricyclic ketone" to afford the corresponding camptothecin-7-ethylamine derivative; 10) substituted 2-acetylphenylamine was condensed with "tricyclic ketone" to afford 7-methylcamptothecin; 11) 7-methylcamptothecin was subjected to a Mannich reaction with the corresponding amine in DMSO to afford the corresponding product.

In one embodiment, the halopropionitrile used in step (1) of synthesis route 1 is chloropropionitrile or bromopropionitrile, and the amount is 1-2 molar equivalents, preferably 1.0-1.5 molar equivalents, and more preferably 1.1-1.2 molar equivalents.

In one embodiment, AlCl₃ or BCl₃ is used for catalysis in step (1) of synthesis route 1, and the amount is 1-3 molar equivalents, preferably 1.0-1.5 molar equivalents.

In one embodiment, PPTS is used for catalysis in step (2) of synthesis route 1, and the amount is 1-2 molar equivalents, preferably 1.0-1.2 equivalents.

In one embodiment, the amount of the amine in step (3) of synthesis route 1 is 1-20 molar equivalents, preferably 3-10 molar equivalents.

In one embodiment, nitration is carried out using nitric acid/acetic anhydride in step (2) of synthesis route 2, and the reaction temperature is -10-10 °C, with a preferred temperature of -5-0 °C.

In one embodiment, the amount of amine in step (2) of synthesis route 4 is 1-20 molar equivalents, preferably 3-6 molar equivalents.

In one embodiment, in step (2) of synthesis method 3, aqueous formaldehyde or paraformaldehyde is used, and the amount is 5-100 molar equivalents, preferably 30-40 molar equivalents.

In one embodiment, the Michael addition reaction in step (3) of synthesis method 3 is carried out at 0-100 °C, preferably at 50-70 °C.

In one embodiment, in the Mannich reaction in step (2) of synthesis method 4, the hydrochloride of primary amine or secondary amine is used and is heated in DMSO at 80-160 °C, preferably at 100-145 °C, and more preferably at 110-140 °C.

In another aspect of the present disclosure, an antibody-drug conjugate is provided, in which the compound or pharmaceutically acceptable salt, stereoisomer or prodrug thereof mentioned above is used as small molecule drug.

In another aspect of the present disclosure, it provides a pharmaceutical composition which comprises the compound or pharmaceutically acceptable salt, stereoisomer, or prodrug thereof or the antibody-drug conjugate mentioned above and a pharmaceutically acceptable excipient.

In another aspect of the present disclosure, it provides a use of the compound or pharmaceutically acceptable salt, stereoisomer, or prodrug thereof, the antibody-drug conjugate or the pharmaceutical composition mentioned above in the manufacture of a medicament for treating a cancer disease.

In another aspect of the present disclosure, it provides a method for treating cancer disease, comprising the step of administering to a patient in need thereof the compound or pharmaceutically acceptable salt, stereoisomer, or prodrug thereof, antibody-drug or pharmaceutical composition mentioned above.

In one embodiment, the amount of the compound or pharmaceutically acceptable salt, stereoisomer or prodrug thereof, antibody-drug conjugate, and pharmaceutical composition mentioned above is a therapeutically effective amount.

In one embodiment, the cancer disease includes gastric cancer, esophageal cancer, cardia cancer, breast cancer, ovarian cancer, colon cancer, rectal cancer, primary liver cancer, acute and chronic granulocytic leukemia, choroidal epithelial cancer, lung cancer, bladder cancer, intestinal cancer and small cell lung cancer; more preferably, the cancer disease is esophageal cancer, breast cancer and gastric cancer.

### DETAILED DESCRIPTION OF THE EMBODIMENTS

### I. Definition

In the present disclosure, unless otherwise specified, the scientific and technical terms used herein have the meanings commonly understood by those skilled in the art. Also, the relevant terms and laboratory procedures used herein are terms and conventional procedures widely used in the corresponding fields. Meanwhile, for better understanding of the present disclosure, definitions and explanations of related terms are provided below.

As used herein and unless otherwise specified, the terms "comprising", "including", "having", "containing", including grammatical equivalents thereof, should generally be understood to be openended and non-limiting, for example, not excluding other unrecited elements or steps.

The compound of the present disclosure can be asymmetric, for example, having one or more stereoisomers. Unless otherwise specified, all stereoisomers are included, for example, enantiomers and diastereomers. The stereoisomers include geometric isomers (such as cis and trans structures) and optical isomers (such as enantiomers), therapeutic substances consisting of monomers, racemates, racemic mixtures and pharmaceutically acceptable salts thereof. The compound of the present disclosure containing asymmetric carbon atoms can be isolated in optically active pure form or racemic form. Optically active pure forms can be resolved from racemic mixtures or synthesized by using chiral starting materials or chiral reagents. Racemates, diastereomers, enantiomers are all included within the scope of the present disclosure.

The compound of the present disclosure also includes tautomeric forms. Tautomeric forms arise from the exchange of one single bond with an adjacent double bond and together with the transfer of one proton.

As used herein, "pharmaceutically acceptable salt" refers to the salt of the corresponding amine compound with an inorganic or organic acid, or the salt of the corresponding carboxylic acid compound with an alkali metal or alkaline earth metal, or with an organic amine. Wherein, inorganic acids include but are not limited to hydrochloric acid, hydrobromic acid, hydroiodic acid, sulfuric acid, phosphoric acid and so on; organic acids include but are not limited to acetic acid, propionic acid, butyric acid, benzoic acid, methanesulfonic acid, toluenesulfonic acid, p-toluenesulfonic acid, oxalic acid, succinic acid, lactic acid, citric acid, succinic acid, gluconic acid, maleic acid, fumaric acid, tartaric acid and so on; alkali metal or alkaline earth metal salts include but are not limited to sodium, potassium, calcium, magnesium salts and so on; organic amine salts include, but are not limited to, salts composed of ammonia, methylamine, ethylamine, propylamine, isopropylamine, dimethylamine, diethylamine, trimethylamine, triethylamine, tert-butylamine, ethylenediamine, ethanolamine, diethanolamine, triethanolamine, morpholine, piperidine, piperazine, amino acids and so on.

As used herein, "prodrug" refers to the form in which a compound is metabolized or simply chemically altered in the body of a patient after the compound has been introduced into the human body in a suitable mode of administration to the compound contained in general formula 1 of the present invention, as well as the corresponding salt. Prodrugs of the compounds include, but are not limited to, various carboxylate esters, carbonate esters, phosphate esters, sulfate esters, sulfonate esters, amino acid esters, gluconate esters, as well as various amides, acetals, hemiacetals, carbamide esters and so on.

The numerical range as used herein refers to each integer within the given range. For example,"C₁-C₆" refers to the group that can have 1 carbon atom, 2 carbon atoms, 3 carbon atoms, 4 carbon atoms, 5 carbon atoms or 6 carbon atoms; "C₃-C₆" refers to the group that can have 3 carbon atoms, 4 carbon atoms, 5 carbon atoms or 6 carbon atoms.

When any variable (such as Rₙ) appears more than once in the composition or structure of a compound, the definition thereof is independent in each case. Therefore, for example, if a group is substituted with 1-5 Rs, then the group can optionally be substituted with up to 5 Rs, and each R has independent options in each case. In addition, combinations of substituents and/or variants thereof are only allowed if such combinations result in the formation of stable compounds.

The term "alkyl" refers to saturated aliphatic hydrocarbon groups, which is a straight or branched chain group containing 1 to 20 carbon atoms, preferably the alkyl containing 1 to 8 carbon atoms, more preferably 1 to 6 carbon atoms, and most preferably 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 2,2-diethylhexyl, 2,2-diethylhexyl, and various branched isomers thereof, etc., more preferably, a lower alkyl containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl can be substituted or unsubstituted, and when substituted, substituents can be present at any available point of attachment, the substituents preferably being one or more of the following groups independently selected from alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group, with methyl, ethyl, isopropyl, tert-butyl, halogenated alkyl, deuterated alkyl, alkoxy-substituted alkyl, and hydroxy-substituted alkyl being preferred for the present disclosure.

The term "heterocyclyl" refers to a saturated or partially unsaturated monocyclic or multicyclic cyclic hydrocarbon substituent containing 3 to 20 ring atoms, wherein one or more ring atoms are heteroatoms selected from nitrogen, oxygen, or S(O)m (wherein m is an integer from 0 to 2), but excluding ring moieties of-O-O-, -O-S-, or-S-S-, with the remaining ring atoms being carbon, preferably containing 3 to 12 ring atoms, of which 1 to 4 are heteroatoms; more preferably containing 3 to 8 ring atoms; most preferably containing 3 to 8 ring atoms; further preferably containing 3 to 8 membered heterocyclic with 1 to 3 nitrogen atoms, optionally substituted with 1 to 2 oxygen atoms, sulfur atoms, oxo, including nitrogen-containing monocyclic heterocyclic group, nitrogen-containing spiroheterocyclic group or nitrogen-containing fused heterocyclic group.

The term "aryl" refers to a 6 to 14 membered all-carbon monocyclic or fused polycyclic (rings sharing adjacent pairs of carbon atoms) group having a conjugated π electron system, preferably 6 to 12 membered, such as phenyl and naphthyl.

Aryl groups can be substituted or unsubstituted. When substituted, the substituents are preferably one or more of the following groups independently selected from: alkyl, alkenyl, alkynyl, alkoxy, alkylthio, alkylamino, halogen, thio, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

The term "alkoxy" refers to -O-(alkyl) and -O-(unsubstituted cycloalkyl), wherein the definition of alkyl is as described above. Non-limiting examples of alkoxy include: methoxy, ethoxy, propoxy, butoxy, cyclopropoxy, cyclobutoxy, cyclopentoxy, cyclohexoxy. Alkoxy can be optionally substituted or unsubstituted, and when substituted, the substituents are preferably one or more of the following groups independently selected from: alkyl, alkenyl, alkynyl, alkoxy, alkylthio, alkylamino, halogen, thio, hydroxy, nitro, chloro, cycloalkyl, heterocycloalkyl, aryl, heteroaryl, cycloalkoxy, heterocycloalkoxy, cycloalkylthio, heterocycloalkylthio, carboxyl, or carboxylate group.

The hydrogen atoms according to the present disclosure can be substituted by the isotope deuterium thereof.

The term "substituted" refers to one or more hydrogen atoms in a group, preferably up to 5, and more preferably 1-3 hydrogen atoms, being independently substituted with the corresponding number of substituents. It goes without saying that the substituents are only in their possible chemical positions, and those skilled in the art can determine (through experiment or theory) which substitutions are possible or impossible without undue effort. For example, combinations such as an amino or hydroxy having a free hydrogen with a carbon atom having an unsaturated (e.g., olefinic) bond may be unstable.

" 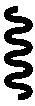 " refers to the chemical bond junction.

### Medicament or pharmaceutical composition

As used herein, "pharmaceutically acceptable salt" refers to the salt of the corresponding amine compound with an inorganic or organic acid, or the salt of the corresponding carboxyllic acid compound with an alkali metal or alkaline earth metal, or with an organic amine. Wherein, inorganic acids include but are not limited to hydrochloric acid, hydrobromic acid, hydroiodic acid, sulfuric acid, phosphoric acid and so on; organic acids include but are not limited to acetic acid, propionic acid, butyric acid, benzoic acid, methanesulfonic acid, toluenesulfonic acid, p-toluenesulfonic acid, oxalic acid, succinic acid, lactic acid, citric acid, succinic acid, gluconic acid, maleic acid, fumaric acid, tartaric acid and so on; alkali metal or alkaline earth metal salts include but are not limited to sodium, potassium, calcium, magnesium salts and so on; organic amine salts include, but are not limited to, salts composed of ammonia, methylamine, ethylamine, propylamine, isopropylamine, dimethylamine, diethylamine, trimethylamine, triethylamine, tert-butylamine, ethylenediamine, ethanolamine, diethanolamine, triethanolamine, morpholine, piperidine, piperazine, amino acids and so on.

As used herein, "prodrug" refers to the form in which a compound is metabolized or simply chemically altered in the body of a patient after the compound has been introduced into the human body in a suitable mode of administration to the compound contained in the general formula 1 of the present invention, as well as the corresponding salt. Prodrugs of the compounds include, but are not limited to, various carboxylate esters, carbonate esters, phosphate esters, sulfate esters, sulfonate esters, amino acid esters, gluconate esters, as well as various amides, acetals, hemiacetals, carbamide esters and so on.

The drugs or pharmaceutical compositions of the present disclosure can be administered orally, topically, parenterally, or mucosally (e.g., buccally, by inhalation, or rectally) in dosage unit formulations comprising conventional non-toxic pharmaceutically acceptable carriers. It is often desirable to use in orally administered route. The active agent can be administered orally in the form of capsules, tablets, etc. (see Remington: The Science and Practice of Pharmacy, 20th Edition).

For oral administration in the form of tablets or capsules, the active pharmaceutical ingredients can be combined with non-toxic, pharmaceutically acceptable excipients such as binders (for example, pregelatinized corn starch, polyvinyl pyrrolidone, or hydroxypropyl methylcellulose); fillers (for example, lactose, sucrose, glucose, mannitol, sorbitol, and other reducing and nonreducing sugars, microcrystalline cellulose, calcium sulfate, or calcium hydrogen phosphate); lubricants (for example, magnesium stearate, talc, or silica, stearic acid, sodium stearyl fumarate, glyceryl behenate, calcium stearate, etc.); disintegrants (for example, potato starch or sodium starch glycolate); or wetting agents (for example, sodium lauryl sulfate), colorants and flavorings, gelatin, sweeteners, natural and synthetic gums (such as acacia gum, tragacanth gum, or alginates), buffering salts, carboxylmethyl cellulose, polyethylene glycol, waxes, etc. For oral administration in liquid form, the pharmaceutical ingredients can be combined with non-toxic, pharmaceutically acceptable inert carriers (for example, ethanol, glycerin, water), anti-sedimentation agents (for example, sorbitol syrup, cellulose derivatives, or hydrogenated edible fats), emulsifiers (for example, lecithin or acacia gum), non-aqueous carriers (for example, almond oil, oil esters, ethanol, or fractionated vegetable oils), preservatives (for example, methyl p-hydroxybenzoate or propyl p-hydroxybenzoate or sorbic acid), etc. Stabilizers such as antioxidants (BHA, BHT, propyl gallate, sodium ascorbate, citric acid) can also be added to stabilize the dosage form.

Tablets containing the active compound can be coated using methods well-known in the field. The compositions of the present disclosure, which include the compound represented by formula I as the active compound, can also be introduced into small beads, microspheres, or microcapsules, for example, constructed with polyglycolic acid/lactic acid (PGLA). Liquid formulations for oral administration can take the forms of e.g. solution, syrup, emulsion, or suspension, or they can be presented as dry products that are reconstituted with water or other suitable excipients before use. Formulations for oral administration can be suitably formulated to provide controlled or delayed release of the active compound.

The term "treatment" includes inhibiting, alleviating, preventing, or eliminating one or more symptoms or side effects associated with the disease, condition, or disorder being treated.

The term "inhibition" is used in relation to controls. Those skilled in the art will readily determine the appropriate control for each experiment. For example, a reduced response in subjects or cells treated with the compound is compared to the response in subjects or cells not treated with the compound.

The term "pharmaceutical composition" refers to a composition that includes the compound or pharmaceutically acceptable salt thereof of the present disclosure, as well as at least one pharmaceutically acceptable component selected from the following, depending on the administration route and the nature of dosage form, including but not limited to: carriers, diluents, adjuvants, excipients, preservatives, fillers, disintegrants, wetting agents, emulsifiers, suspension agents, sweeteners, flavoring agents, fragrances, antimicrobials, antifungals, lubricants, dispersing agents, temperature-sensitive materials, temperature regulators, adhesives, stabilizers, and suspending agents.

The term "effective amount" or "therapeutically effective amount" refers to a sufficient amount of a drug or medication that is non-toxic but can achieve the desired effect. In embodiments of the present disclosure, when treating a patient in accordance with the present disclosure, the amount of drugs administered depends on many factors, such as the specific dosing regimen, the type of disease or condition and the severity thereof, the uniqueness (for example, weight) of the treated person or host to be treated, but the amount of drug administered depends on specific surrounding circumstances, including, for example, the specific drug that has been employed, the route of administration, the treated condition, and the treated person or host to be treated, the administered dose may be routinely determined by methods known in the art. Generally, regarding the dosage used for adult therapy, the administered dose is typically within the range of 0.02-5000 mg/day, for example, the range of about 1-1500 mg/day. The required dose can be conveniently administered as a single dose, or as doses given simultaneously (or in a short period of time) or in divided doses at appropriate intervals, such as two, three, four doses per day or more. It can be understood by those skilled in the art that, although the above dose ranges are provided, the specific effective amount can be appropriately adjusted based on the patient's condition and in conjunction with a physician's diagnosis.

The term "antibody-drug conjugate (ADC)" refers to a biopharmaceutical product that composes a small molecule drug linked to a monoclonal antibody through a chemical linker, in which the monoclonal antibody is used as a carrier to transport the small molecule drug to its target cells.

As used herein, the terms "reduce", "inhibit", "alleviate", or "decrease" are used in relation to controls. Those skilled in the art will easily determine the appropriate control for each experiment. For example, a reduced response in subjects or cells treated with the compound is compared to the response in subjects or cells not treated with the compound.

Unless otherwise specified, the raw materials and equipment used in the specific embodiments of the present disclosure are known products that can be obtained by purchasing commercial products.

### II. Examples

The screening method of the present disclosure included: tests for the growth inhibitory activities of compounds against tumor cells such as OE33 cells (human esophageal cancer cells), SKBR3 cells (human breast adenocarcinoma cells), etc.

### Example 1: 7-(2-Acetylamino)ethyl-10-methyl-11-fluorocamptothecin (1)

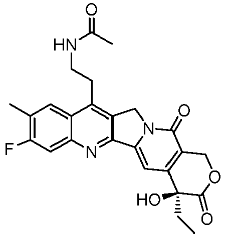

To a single-neck flask containing 160 mL of anhydrous 1,2-dichloroethane, a 1M solution of BCl₃ in dichloromethane (32 mL, 0.032 mol) were added. The mixture was cooled to 0°C, and then 3-fluoro-4-methylaniline (5.0 g, 0.04 mmol) was added. The mixture was reacted at 0°C for 10min, then acetonitrile (16.40 g, 0.4 mol) and aluminum trichloride (7 g, 0.05 mol) were added. The mixture was slowly warmed to room temperature and stirred for 10min, then warmed to 80°C, and stirred for 12h. After cooling, the reaction liquid was poured into ice water, and a 1M solution of HCl was added to adjust the pH=2. The mixture was extracted with dichloromethane, dried over Na₂SO₄, and purified by silica gel column chromatography to afford the intermediate 6-amino-4-fluoro-3-methylacetophenone (2.5g, yield 37.4%, HPLC 94%); LC-MS (M+H)⁺ 168.02 (calculated value 167.07).

6-Amino-4-fluoro-3-methylacetophenone (1.5 g, 9.0 mmol) was taken and dissolved in anhydrous toluene, then "tricyclic ketone" (2.36 g, 9.0 mmol) and PPTS (0.6 g, 2.4 mmol) were added. The reaction was heated to 115°C and stirred for 12h. The solvent was removed by concentrating under reduced pressure, and 10 mL of methanol was added. The mixture was filtered, and the filter cake was dried to afford 7,10-dimethyl-11-fluorocamptothecin (3.2 g, yield 90.4%, HPLC 96%); LC-MS (M+H)⁺ 395.26 (calculated value 394.13).

127.1 Mg of amine hydrochloric and 148 µL of hydrochloric acid were added to 1.5 mL of DMSO, and the reaction was warmed to 110°C in an oil bath pan, then 7,10-dimethyl-11-fluorocamptothecin (100 mg, 0.25 mmol) was added. The mixture was warmed to 130-140°C and reacted for 50 minutes, then cooled to room temperature, then methanol was added. The mixture was filtered and purified by silica gel column chromatography to afford the demethoxy product, 7-(2-amino)ethyl-10-methyl-11-fluorocamptothecin (41 mg, yield 38.7%, HPLC 94%); ¹H NMR (500 MHz, DMSO-d₆) δ 8.02-7.92 (m, 3H), 7.35 (d, J=2.6 Hz, 1H), 6.61-6.53 (m, 1H), 5.43 (d, J=33.0 Hz, 4H), 3.59-3.47 (m, 2H), 3.22 (s, 2H), 1.89 (dt, *J=14.5,* 6.9 Hz, 2H), 0.89 (q, *J*=5.3 Hz, 3H); LC-MS (M+H)⁺ 424.02 (calculated value 423.16).

7-(2-Amino)ethyl-10-methyl-11-fluorocamptothecin (10 mg, 0.023 mmol) was added to a 25 mL single-neck flask, DMF (5 mL) was added, and acetic acid (2.8 mg, 0.046 mmol) was added, then HATU (18 mg, 0.046 mmol) and DIPEA (6 mg, 0.046 mmol) were added. The mixture was reacted at room temperature for 1 hour, then quenched by adding 0.5 mL of water dropwise. The reaction liquid was prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the product 7-(2-acetylamino)ethyl-10-methyl-11-fluorocamptothecin (4.78 mg, yield 45.5%, HPLC 97.5%); ¹H NMR (500 MHz, DMSO-d₆) δ 8.32 (d, *J*=8.0 Hz, 1H), 7.90 (d, *J=10.5* Hz, 1H), 7.30 (s, 1H), 5.44 (s, 3H), 5.41 (s, 2H), 5.31 (s, 2H), 2.47 (s, 2H), 2.30 (s, 2H), 1.90 (d, J=7.1 Hz, 3H), 1.85 (dt, *J=14.1,* 7.5 Hz, 2H), 0.89 (d, J=7.1 Hz, 3H); LC/MS (M+H)⁺ 466.41 (calculated value 465.17).

### Example 2: 7-(2-Difluoroacetylamino)ethyl-10-methyl-11-fluorocamptothecin (2)

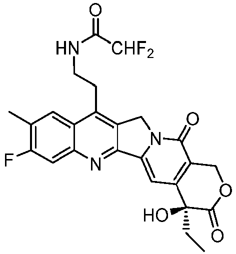

7-(2-Amino)ethyl-10-methyl-11-fluorocamptothecin (15 mg, 0.035 mmol) was added to a reaction flask, DMF (5 mL) was added, difluoroacetic acid (8.8 mg, 0.092 mmol) was added, then HATU (18 mg, 0.046 mmol) and DIPEA (6 mg, 0.046 mmol) were added. The mixture was reacted at room temperature for 1 hour, then quenched by adding 0.5 mL of water dropwise. The reaction liquid was prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the product 7-(2-difluoroacetylamino)ethyl-10-methyl-11-fluorocamptothecin (7.29 mg, yield 41.1%, HPLC 96 %); ¹H NMR (500 MHz, DMSO-d₆) δ 8.34 (d, J=8.1 Hz, 1H), 7.93 (d, J=10.6 Hz, 1H), 7.31 (s, 1H), 6.53 (d, J=4.9 Hz, 1H), 5.44 (s, 3H), 5.42 (s, 2H), 5.34 (s, 2H), 2.53 (s, 2H), 2.30 (s, 1H), 1.88 (s, 2H), 0.89 (d, J=7.3 Hz, 3H); LC/MS (M+H)⁺ 502.28 (calculated value 501.46).

### Example 3: 7-(2-Methylsulfonylamino)ethyl-10-methyl-11-fluorocamptothecin (3)

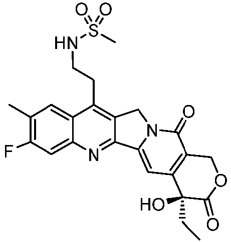

7-(2-Amino)ethyl-10-methyl-11-fluorocamptothecin (10 mg, 0.023 mmol, Example 1) was added to a reaction flask, DMF (5 mL) was added, then methanesulfonyl chloride (4 mg, 0.035 mmol) and DIPEA (6 mg, 0.046 mmol) were added. The mixture was reacted at room temperature for 1 hour, then quenched by adding 0.5 mL of water dropwise. The reaction liquid was prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the product 7-(2-methylsulfonylamino)ethyl-10-methyl-11-fluorocamptothecin (6.22 mg, yield 54 %, HPLC 97 %); LC/MS (M+H)⁺ 502.23 (calculated value 501.53).

### Example 4: 7-(2-(Tetrahydropyran-4-yl)amino)ethyl-10-methyl-11-fluorocamptothecin (4)

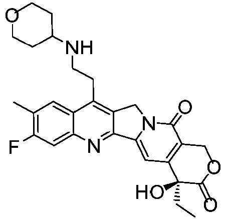

To a reaction flask, 4-aminotetrahydropyran (154 mg, 1.52 mmol), 7,10-dimethyl-11-fluorocamptothecin (100 mg, 0.25 mmol), hydrochloric acid (0.138 mL, 1.65 mmol), and DMSO (1 mL) were added respectively. The mixture was heated to 120-130 °C under stirring for 40 minutes. After cooling, isopropanol was added. The mixture was filtered and purified by silica gel column chromatography to afford the compound 7-(2-(tetrahydropyran-4-yl)amino)ethyl-10-methyl-11-fluorocamptothecin (49.3 mg, yield 39%, HPLC 95%); ¹H NMR (500 MHz, DMSO-d₆) δ 8.01 (d, J=8.6 Hz, 1H), 7.83 (s, 1H), 7.35 (s, 1H), 6.61 (s, 1H), 5.77 (s, 3H), 5.45 (d, J=22.3 Hz, 4H), 3.98-3.89 (m, 2H), 3.57-3.52 (m, 2H), 3.34-3.27 (m, 5H), 1.96 (d, J=10.6 Hz, 2H), 1.88 (dd, J=12.1, 7.3 Hz, 2H), 1.56 (d, J=9.7 Hz, 2H), 0.88 (t, J=7.3 Hz, 3H); LC-MS (M+H)⁺ 508.33 (calculated value 507.22).

### Example 5: 7-(2-(piperazin-4-yl))ethyl-10-methyl-11-fluorocamptothecin (5)

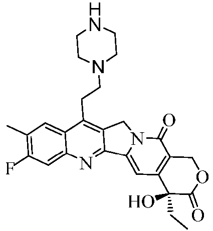

To a reaction flask, piperazine (86.14mg, 1 mmol), hydrochloric acid (0.085 mL, 1.02 mmol) and DMSO (1 mL) were added. The mixture was warmed to 110 °C under stirring, then 7,10-dimethyl-11-fluorocamptothecin (100 mg, 0.25 mmol) was added. The mixture was continued to stir at 120-135°C and reacted for 1h. After cooling, isopropanol was added. The mixture was filtered and purified by silica gel column chromatography to afford the compound 7-(2-(piperazin-4-yl))ethyl-10-methyl-11-fluorocamptothecin (43.75 mg, yield 35.5%, HPLC 98%); ¹H NMR (500 MHz, DMSO-d₆) δ 8.21 (d, J=8.1 Hz, 1H), 7.89 (d, J=10.7 Hz, 1H), 7.31 (s, 1H), 6.55 (s, 1H), 5.44 (s, 2H), 5.36 (s, 2H), 3.62 (s, 6H), 3.44 (s, 3H), 3.24 (s, 4H), 2.52 (s, 2H), 1.87 (dp, J=21.6, 7.1 Hz, 2H), 0.88 (t, *J*=7.3 Hz, 3H); LC-MS (M+H)⁺ 493.15 (calculated value 492.22).

### Example 6: 7-(2-(4-Hydroxycyclohexyl)amino)ethyl-10-methyl-11-fluorocamptothecin (6)

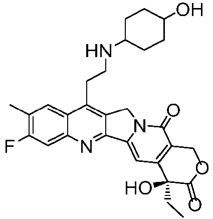

Trans-4-Aminocyclohexanol (175.22mg, 1.52 mmol), hydrochloric acid (0.138 mL, 1.65 mmol) and DMSO (1 mL) were added to a reaction flask. The mixture was warmed to 110°C under stirring, then 7,10-dimethyl-11-fluorocamptothecin (100 mg, 0.25 mmol) was added. The mixture was warmed to 120-130°C and continued to react for 45 minutes, then cooled to room temperature, then methyl tert-butyl ether was added. The mixture was filtered and purified by silica gel column chromatography to afford the compound 7-(2-(4-hydroxycyclohexyl)amino)ethyl-10-methyl-11-fluorocamptothecin (54.8 mg, yield 42.0%, HPLC 97%); ¹H NMR (500 MHz, DMSO-d₆) δ 8.85 (d, J=63.5 Hz, 2H), 8.15 (dd, *J=117.3,* 8.3 Hz, 1H), 7.98-7.79 (m, 1H), 7.38 (d, J=6.1 Hz, 1H), 6.62 (s, 1H), 5.51 (d, J=3.9 Hz, 2H), 5.41 (d, *J*=8.8 Hz, 2H), 3.64-3.41 (m, 7H), 3.26-3.09 (m, 1H), 2.54-2.51 (m, 1H), 2.10 (d, *J=10.0* Hz, 1H), 2.00-1.87 (m, 3H), 1.85-1.73 (m, 2H), 1.47 (dq, J=24.7, 12.9 Hz, 2H), 1.25 (td, *J=13.2,* 12.8, 6.9 Hz, 1H), 1.01-0.87 (m, 3H); LC-MS (M+H)⁺ 522.15 (calculated value 521.23).

### Example 7: 7-(2-(4-Methoxycyclohexyl)amino)ethyl-10-methyl-11-fluorocamptothecin (7)

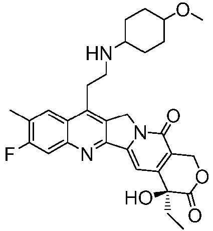

To a reaction flask, trans-4-methoxycyclohexylamine (196.56 mg, 1.52 mmol), hydrochloric acid (0.138 mL, 1.65mmol), 7,10-dimethyl-11-fluorocamptothecin (100 mg, 0.25 mmol) and DMSO (1 mL) were added respectively. The mixture was warmed to 120-130 °C under stirring and reacted for 45 minutes. After cooling, isopropanol was added. The mixture was filtered and purified by silica gel column chromatography to afford the compound 7-(2-(4-methoxycyclohexyl)amino)ethyl-10-methyl-11-fluorocamptothecin (49.6 mg, yield 37.0%, HPLC 97%); ¹H NMR (500 MHz, DMSO-d₆) δ 8.71 (s, 2H), 8.23-8.14 (m, 1H), 8.00 (d, J=8.6 Hz, 1H), 7.82 (s, 1H), 7.35 (s, 1H), 5.46 (s, 2H), 5.41 (s, 2H), 3.81-3.73 (m, 1H), 3.24 (s, 3H), 3.12 (q, *J*=10.4 Hz, 3H), 2.63 (s, 2H), 2.28 (s, 2H), 1.95-1.81 (m, 4H), 1.37 (d, *J=*11.0 Hz, 4H), 0.88 (t, *J*=7.4 Hz, 3H); LC-MS (M+H)⁺ 536.41 (calculated value 535.25).

### Example 8: 7-(2-(2-Azidoethyl)amino)ethyl-10-methyl-11-fluorocamptothecin (8)

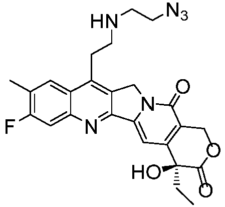

To a reaction flask, 2-azidoethanamine hydrochloride (218.30 mg, 2.54 mmol), 7,10-dimethyl-11-fluorocamptothecin (500mg, 1.27 mmol) and DMSO (10 mL) were added. The mixture was warmed to 120-135°C under stirring and reacted for 50 minutes, then cooled to room temperature, then methyl tert-butyl ether was added. The mixture was filtered and purified by silica gel column chromatography to afford the compound 7-(2-(2-azidoethyl)amino)ethyl-10-methyl-11-fluorocamptothecin (294 mg, yield 47%, HPLC 95%); ¹H NMR (600 MHz, DMSO-d₆) δ 8.33 (d, J=8.1 Hz, 1H), 8.24 (s, 2H), 7.90 (d, J=10.6 Hz, 1H), 7.30 (s, 1H), 6.55 (s, 1H), 5.41 (s, 2H), 5.31 (s, 2H), 3.66 (dd, *J=12.9,* 4.7 Hz, 2H), 3.54 (dd, *J=12.9,* 6.6 Hz, 2H), 2.47 (d, J=2.2 Hz, 2H), 2.30 (s, 2H), 1.90-1.86 (m, 2H), 1.20 (d, J=6.7 Hz, 3H), 0.88 (s, 3H); LC-MS (M+H)⁺ 493.21 (492.19).

### Example 9: 7-(2-((S)-4-Hydroxybutan-2-yl)amino)ethyl-10-methyl-11-fluorocamptothecin (9)

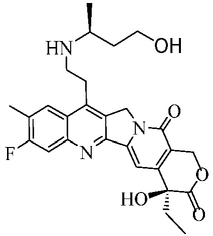

To a reaction flask, (R)-3-aminobutanol (135.60mg, 1.52 mmol), hydrochloric acid (0.138 mL, 1.65 mmol) and DMSO (1 mL) were added. The mixture was warmed to 110°C under stirring, then 7,10-dimethyl-11-fluorocamptothecin (100 mg, 0.25 mmol) was added. The mixture was warmed to 130-140°C and continued to react for 50 minutes. After cooling, isopropanol was added. The mixture was filtered and purified by silica gel column chromatography to afford the compound 7-(2-((S)-4-hydroxybutan-2-yl)amino)ethyl-10-methyl-11-fluorocamptothecin (53.4 mg, yield 43.1%, HPLC 99%); ¹H NMR (500 MHz, DMSO-d₆) δ 8.77 (d, J=68.4 Hz, 1H), 8.25 (d, J=8.0 Hz, 1H), 7.91 (d, J=10.7 Hz, 1H), 7.32 (s, 1H), 6.55 (s, 1H), 5.40 (d, J=51.6 Hz, 4H), 3.84-3.70 (m, 2H), 3.54-3.50 (m, 2H), 3.27 (s, 2H), 3.14-3.07 (m, 2H), 2.53 (s, 3H), 1.87 (dq, *J*=21.6, 7.2 Hz, 2H), 1.64 (dt, *J=13.6,* 6.7 Hz, 1H), 1.26 (d, *J*=6.4 Hz, 3H), 0.88 (t, *J=7.2* Hz, 3H); LC-MS (M+H)⁺ 496.36 (calculated value 495.22).

### Example 10: 7-(2-Hydroxyethylamino)ethyl-10,11-difluorocamptothecin (10)

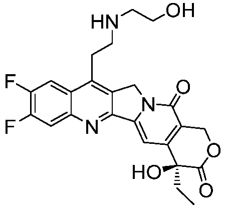

To a single-neck flask containing anhydrous 1,2-dichloroethane (80 mL), a 1M solution of BCl₃ in dichloromethane (16 mL, 0.016 mol) were added. The reaction flask was cooled to 0°C, then 3,4-difluoroaniline (2.5g, 0.019 mol) was added. The mixture was reacted at 0°C for 10min, then acetonitrile (8.2 g, 0.2 mol) and aluminum trichloride (3.5 g, 0.025 mol) were added. The mixture was slowly warmed to room temperature and stirred for 10 minutes, then warmed to 80°C, and stirred for 12h. The reaction liquid was poured into ice water, and a 1M solution of HCl was added to adjust the pH=2. The mixture was extracted with dichloromethane, dried over Na₂SO₄. Then silica gel powder was added and the mixture was purified by column chromatography (PE:EA=100%-80%) to afford 6-amino-3,4-difluoroacetophenone (1.0 g, yield 30.2%, HPLC 94%); LC-MS (M+H)⁺ 172.21 (calculated value 171.05).

6-Amino-3,4-difluoroacetophenone (0.50g, 2.9 mmol) was taken and dissolved in anhydrous toluene (5 mL), then "tricyclic ketone" (769.07 mg, 2.9 mmol) and PPTS (73mg, 0.29 mmol) were added. The reaction was heated to 115°C and stirred for 12 hours. After cooling, the solvent was removed under reduced pressure, and 5 mL of methanol was added. The mixture was filtered and dried to afford 7-methyl-10,11-difluorocamptothecin (0.82g, yield 70.69%, HPLC 96%); LC-MS (M+H)⁺ 399.23 (calculated value 398.11).

To a reaction flask, ethanolamine (46 mg, 0.75 mmol), hydrochloric acid (0.07 mL, 0.8 mmol) and DMSO (1 mL) were added. The mixture was warmed to 120°C under stirring, then 7-methyl-10,11-difluorocamptothecin (50 mg, 0.125 mmol) was added. The mixture was reacted at 120-140°C for 30 minutes, then cooled to room temperature, then methyl tert-butyl ether was added. The mixture was filtered and purified by silica gel column chromatography to afford the product 7-(2-hydroxyethylamino)ethyl-10,11-difluorocamptothecin (31 mg, yield 52.5%, HPLC 98%): ¹H NMR (500 MHz, DMSO-d₆) δ 8.64 (s, 2H), 8.39 (dd, *J=*11.9, 8.6 Hz, 1H), 8.29 (dd, *J=11.3,* 8.1 Hz, 1H), 7.35 (s, 1H), 6.58 (s, 1H), 5.46 (s, 2H), 5.41 (s, 3H), 3.69 (s, 2H), 3.58-3.49 (m, 2H), 3.30 (d, *J=5.4* Hz, 2H), 3.10 (s, 2H), 1.88 (dt, *J*=18.5, 7.0 Hz, 2H), 0.88 (t, J=7.3 Hz, 3H): LC-MS (M+H)⁺ 472.09 (calculated value 471.16).

### Example 11: 7-(2-((S)-2-Methoxyisopropyl)amino)ethyl-10,11-difluorocamptothecin (11)

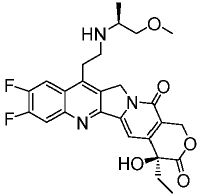

To a reaction flask, (S)-1-methoxy-2-propylamine (100 mg, 1.02 mmol), hydrochloric acid (0.09 mL, 1.08 mmol) and DMSO (1.5 mL) were added. The mixture was warmed to 120°C under stirring, then 7-methyl-10,11-difluorocamptothecin (50 mg, 0.125 mmol) was added. The mixture was reacted at 120-140°C for 35 minutes, then cooled to room temperature, then methyl tert-butyl ether was added. The mixture was filtered, and purified by silica gel column chromatography to afford the compound 7-(2-((S)-2-methoxyisopropyl)amino)ethyl-10,11-difluorocamptothecin (42 mg, yield 67%, HPLC 98%); ¹H NMR (500 MHz, DMSO-d₆) δ 8.66 (s, 1H), 8.37 (dd, *J=11.9,* 8.6 Hz, 1H), 8.30 (dd, *J=11.3,* 8.1 Hz, 1H), 7.36 (s, 1H), 6.58 (s, 1H), 5.46 (s, 2H), 5.41 (d, *J*=4.5 Hz, 2H), 3.84-3.44 (m, 6H), 3.32-3.25 (m, 4H), 1.88 (dd, *J=11.6,* 7.3 Hz, 2H), 1.24 (d, *J*=6.4 Hz, 3H), 0.88 (t, *J=7.2* Hz, 3H); LC-MS (M+H)⁺ 500.39 (calculated value 499.19).

### Example 12.: 7-(2-(4-Hydroxycyclohexyl)amino)ethyl-10,11-difluorocamptothecin (12)

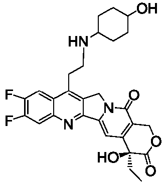

4-Aminocyclohexanol (87 mg, 0.75 mmol) and hydrochloric acid (0.07 mL, 0.84 mmol) were added to a reaction flask containing DMSO (1mL). The mixture was warmed to 110°C under stirring, then 7-methyl-10,11-difluorocamptothecin (50 mg, 0.125 mmol) was added. The mixture was reacted at 120-140°C for 40 minutes. After cooling to room temperature, isopropanol was added. The mixture was purified by silica gel column chromatography to afford the compound 7-(2-(4-hydroxycyclohexyl)amino)ethyl-10,11-difluorocamptothecin (33 mg, yield 50%, HPLC 97%); ¹H NMR (500 MHz, DMSO-d₆) δ 8.71 (s, 1H), 8.63 (s, 1H), 8.34 (dd, *J*=11.6*,* 8.9 Hz, 1H), 8.27 (dd, J=11.2, 8.1 Hz, 1H), 7.34 (s, 1H), 6.57 (s, 1H), 5.46 (s, 2H), 5.40 (s, 2H), 3.81 (s, 1H), 3.52-3.48 (m, 2H), 3.29 (d, *J=*4.4 Hz, 2H), 3.19-3.09 (m, 1H), 1.89 (ddq, *J=21.4,* 14.1, 7.1 Hz, 2H), 1.73 (dd, *J*=18.8, 9.0 Hz, 4H), 1.45 (t, *J*=14.5 Hz, 2H), 1.37 (d, *J*=12.3 Hz, 1H), 1.21 (dd, *J*=18.7, 8.4 Hz, 1H), 0.88 (t, *J*=7.3 Hz, 3H); LC-MS (M+H)⁺ 526.15 (calculated value 525.21).

### Example 13: 7-(2-(Tetrahydropyran-4-yl)amino)ethyl-10,11-difluorocamptothecin (13)

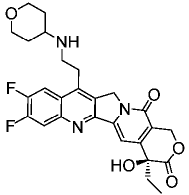

To a reaction flask, 4-aminotetrahydropyran (88 mg, 0.86 mmol), hydrochloric acid (0.072mL, 0.86 mmol), 7-methyl-10,11-difluorocamptothecin (50 mg, 0.125 mmol) and DMSO (1.5 mL) were added. The mixture was heated for 30 minutes to 135-145°C under stirring, then methyl tert-butyl ether was added. The mixture was filtered and then purified by silica gel column chromatography to afford the compound 7-(2-(tetrahydropyran-4-yl)amino)ethyl-10,11-difluorocamptothecin (43 mg, yield 67%, HPLC 95%); ¹H NMR (500 MHz, DMSO-d₆) δ 8.68 (s, 2H), 8.32 (ddd, *J*=19.2, 11.4, 8.4 Hz, 2H), 7.35 (s, 1H), 6.57 (s, 1H), 5.44 (d, *J*=18.6 Hz, 4H), 3.93 (dd, *J*=11.2, 3.5 Hz, 2H), 3.50 3.47 (m, 3H), 3.31 (t, *J*=11.6 Hz, 4H), 1.94 (d, *J*=11.7 Hz, 2H), 1.87 (dd, *J*=15.5, 7.5 Hz, 2H), 1.56 (d, *J*=12.0 Hz, 2H), 0.87 (t, *J*=7.3 Hz, 3H); LC-MS (M+H)⁺ 512.09 (calculated value 511.19).

### Example 14: 7-(2-(4-Methoxycyclohexyl)amino)ethyl-10,11-difluorocamptothecin (14)

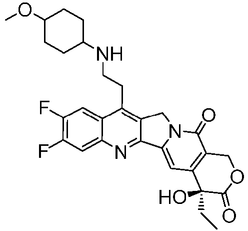

To a reaction flask, 4-methoxycyclohexylamine (0.12 g, 0.9 mmol), hydrochloric acid (0.075 mL, 0.9 mmol) and DMSO (1 mL) were added. The mixture was warmed to 120 °C under stirring, then 7-methyl-10,11-difluorocamptothecin (50 mg, 0.125 mmol) was added. The mixture was warmed to 130°C, then reacted for 30 minutes, then cooled to room temperature, then methanol was added. The mixture was filtered by suction and purified by silica gel column chromatography to afford the compound 7-(2-(4-methoxycyclohexyl)amino)ethyl-10,11-difluorocamptothecin (35.2 mg, yield 55.8%, HPLC 99%); ¹H NMR (500 MHz, DMSO-d₆) δ 8.54 (s, 1H), 8.32 (ddd, *J*=21.8, 11.6, 8.3 Hz, 1H), 7.36 (s, 1H), 6.58 (s, 1H), 5.44 (d, *J*=19.1 Hz, 4H), 3.48 (s, 2H), 3.28 (d, *J*=4.7 Hz, 2H), 3.24 (s, 3H), 3.14-3.05 (m, 2H), 2.07 (d, *J=*10.5 Hz, 4H), 1.88 (dt, *J=*18.8, 7.0 Hz, 2H), 1.37 (dd, *J*=23.3, 11.8 Hz, 2H), 1.16 (dd, *J*=23.0, 10.6 Hz, 2H), 0.88 (t, *J*=7.3 Hz, 3H); LC-MS (M+H)⁺ 540.30 (calculated value 539.22).

### Example 15: 7-(2-(2-Azidoethyl)amino)ethyl-10,11-difluorocamptothecin (15)

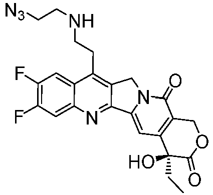

To a reaction flask, 2-azidoethanamine hydrochloride (80mg, 0.93 mmol), 7-methyl-10,11-difluorocamptothecin (50 mg, 0.125 mmol) and DMSO (1 mL) were added. The mixture was warmed to 120-140°C under stirring for 30 minutes, then cooled to room temperature, then isopropanol was added. The mixture was filtered and purified by silica gel column chromatography to afford the product 7-(2-(2-azidoethyl)amino)ethyl-10,11-difluorocamptothecin (39 mg, yield 63%, HPLC 94%); ¹H NMR (500 MHz, DMSO-d₆) δ 8.77 (s, 1H), 8.37 (dd, *J=11.9,* 8.6 Hz, 1H), 8.30 (dd, *J=11.4,* 8.1 Hz, 1H), 7.36 (s, 1H), 6.58 (s, 1H), 5.46 (s, 2H), 5.41 (s, 2H), 3.97-3.71 (m, 2H), 3.53 (d, *J=4.2* Hz, 2H), 3.37-3.26 (m, 2H), 3.21 (s, 2H), 1.88 (dt, J=18.5, 7.0 Hz, 2H), 0.88 (t, J=7.3 Hz, 3H); LC-MS (M+H)⁺ 497.13 (calculated value 496.17).

### Example 16: 7-(2-(2-Azidoethyl)amino)ethyl-10,11-methylenedioxycamptothecin (16)

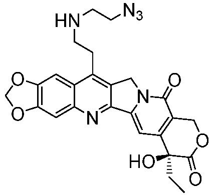

3,4-Methylenedioxy-6-aminoacetophenone (5 g, 0.028 mol) was dissolved in anhydrous toluene (10 mL), then "tricyclic ketone" (7.35 g, 0.028 mol) and PPTS (0.7 g, 0.0028 mol) were added. The reaction was heated to 115°C and stirred for 12h, then cooled. The solvent was removed by concentrating under reduced pressure, and 20 mL of methanol was added. The mixture was filtered and dried to afford 7-methyl-10,11-methylenedioxycamptothecin (11.0 g, yield 97%); LC-MS (M+H)⁺ 407.15 (calculated value 406.12).

2-Azidoethanamine hydrochloride (63.56 mg, 0.74 mmol), DMSO (1 mL) and 7-methyl-10,11-methylenedioxycamptothecin (50 mg, 0.12 mmol) were warmed to 110-130°C under stirring and reacted for 1h, then isopropanol was added. The solid was filtered out and purified by silica gel column chromatography to afford the product 7-(2-(2-azidoethyl)amino)ethyl-10,11-methylenedioxycamptothecin (29 mg, yield 47.9%, HPLC 99%); ¹H NMR (500 MHz, DMSO-d₆) δ 8.91 (s, 2H), 7.61 (s, 1H), 7.48 (s, 1H), 7.20 (s, 1H), 6.26 (d, *J=2.4* Hz, 1H), 5.39 (s, 1H), 5.22 (s, 1H), 3.75-3.69 (m, 2H), 3.18 (d, *J*=26.4 Hz, 4H), 2.47-2.44 (m, 2H), 1.83 (dt, *J*=14.3, 6.9 Hz, 2H), 0.83 (t, *J*=7.3 Hz, 3H); ¹³C NMR(126 MHz, DMSO) δ 163.00, 147.31,141.50, 140.63, 140.00, 137.65, 136.58, 127.48, 118.93, 118.93,108.67, 96.12, 93.28, 89.64, 86.54, 62.88, 55.70, 40.28, 37.46, 36.36, 36.36, 30.86, 20.71, 16.74; LC-MS (M+H)⁺ 505.17 (calculated value 504.18).

### Example 17: 7-(2-(2-Methoxyethyl)amino)ethyl-10,11-methylenedioxycamptothecin (17)

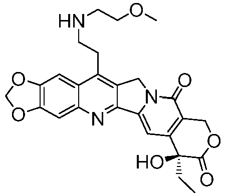

2-Methoxyethylamine (2.5 g, 0.03 mol) and hydrochloric acid (4 mL, 0.05 mol) were dissolved in DMSO (10 mL). The mixture was heated to 110°C under stirring, then 7-methyl-10,11-methylenedioxycamptothecin (1.8 g, 4.4 mmol) was added. The mixture was continued to warm to 120-130°C and react for 1h, then cooled to room temperature, then methyl tert-butyl ether was added. The mixture was filtered and purified by silica gel column chromatography to afford the compound 7-(2-(2-methoxyethyl)amino)ethyl-10,11-methylenedioxycamptothecin (739 mg, yield 34%, HPLC 97%); ¹H NMR (500 MHz, DMSO-d₆) δ 7.55 (s, 1H), 7.44 (s, 1H), 7.20 (s, 1H), 6.51 (s, 1H), 6.27 (d, J=3.6 Hz, 2H), 5.41 (d, J=4.6 Hz, 2H), 5.13 (q, J=18.6 Hz, 2H), 3.31-3.18 (m, 7H), 2.94 (t, J=7.5 Hz, 2H), 2.85 (d, J=4.9 Hz, 2H), 1.86 (dt, J=19.4, 7.0 Hz, 2H), 0.88 (t, J=7.3 Hz, 3H); LC-MS (M+H)⁺ 494.12 (calculated value 493.18).

### Example 18: 7-(2-((S)-4-Hydroxybutyl-2-yl)amino)ethyl-10,11-methylenedioxycamptothecin (18)

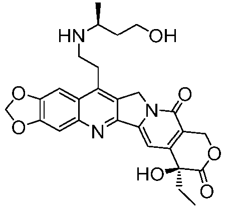

(S)-3-Aminobutanol (146.7 mg, 1.65 mmol), concentrated hydrochloric acid (0.14 mL, 1.68 mmol), DMSO (5 mL) and 7-methyl-10,11-methylenedioxycamptothecin (100 mg, 0.24 mmol) were added to a reaction flask. The reaction mixture was heated to 110-125°C under stirring and reacted for 50 minutes, and cooled to room temperature, then methyl tert-butyl ether was added. The precipitated solid was filtered out and purified by silica gel column chromatography to afford the compound 7-(2-((S)-4-Hydroxybutyl-2-yl)amino)ethyl-10,11-methylenedioxycamptothecin (62 mg, yield 51%, HPLC 93%); ¹H NMR (500 MHz, DMSO-d₆) δ 11.53 (s, 1H), 8.45 (d, *J*=7.7 Hz, 1H), 7.57 (s, 1H), 7.46 (s, 1H), 7.31 (d, *J*=7.7 Hz, 2H), 6.93 (s, 2H), 6.28 (s, 2H), 5.31 (s, 2H), 4.03 (s, 2H), 3.56-3.48 (m, 3H), 3.43 (d, *J*=4.5 Hz, 3H), 1.87-1.71 (m, 2H), 1.66 (dd, *J=*13.4*,* 6.7 Hz, 1H), 1.52 (dd, *J*=13.4, 6.4 Hz, 1H), 1.10 (d, J=6.5 Hz, 3H), 0.84 (t, *J*=7.3 Hz, 3H); LC-MS (M+H)+ 508.33 (calculated value 507.20).

### Example 19: 7-(2-((S)-2-Methoxyisopropyl)amino)ethyl-10,11-methylenedioxycamptothecin (19)

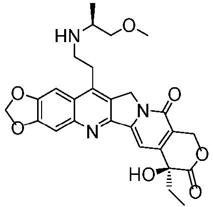

To a reaction flask, (S)-1-methoxy-2-propylamine (100 mg, 1.12 mmol), hydrochloric acid (0.7 mL, 0.8 mmol), DMSO (3 mL) and 7-methyl-10,11-methylenedioxycamptothecin (50 mg, 0.12 mmol) were warmed to 120°C under stirring and reacted for 50 minutes, and cooled to room temperature, then methyl tert-butyl ether was added. The precipitated solid was filtered out and purified by silica gel column chromatography to afford the product 7-(2-((S)-2-methoxyisopropyl)amino)ethyl-10,11-methylenedioxycamptothecin (36 mg, yield 61%, HPLC 99%); ¹H NMR (500 MHz, DMSO-d₆) δ 8.64 (s, 2H), 7.69 (s, 1H), 7.57 (s, 1H), 7.27 (s, 1H), 6.53 (s, 1H), 6.33 (s, 2H), 5.44 (s, 1H), 5.33 (d, *J=4.7* Hz, 2H), 3.71-3.54 (m, 2H), 3.47 (dd, *J=10.1,* 5.8 Hz, 6H), 3.25 (d, J=13.6 Hz, 2H), 1.88 (dt, *J=14.2,* 9.2 Hz, 2H), 1.24 (d, J=6.4 Hz, 3H), 0.88 (dd, J=9.4, 5.3 Hz, 3H); LC-MS (M+H)⁺ 508.37 (calculated value 507.20).

### Example 20: 7-(2-(3-Oxetanyl)amino)ethyl-10,11-methylenedioxycamptothecin (20)

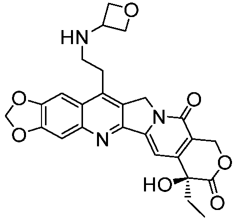

3-Oxetanamine (84 mg, 1.14 mmol) and hydrochloric acid (0.1 mL, 1.2 mmol) were dissolved in DMSO (2 mL). The mixture was heated to 120°C under stirring, then 7-methyl-10,11-methylenedioxycamptothecin (100 mg, 0.24 mmol) was added. The mixture was warmed to 130°C and reacted for 50 minutes. After cooling, methyl tert-butyl ether was added. The mixture was filtered and purified by silica gel column chromatography to afford the compound 7-(2-(3-oxetanyl)amino)ethyl-10,11-methylenedioxycamptothecin (49 mg, yield 41.5%, HPLC 94%); ¹H NMR (500 MHz, DMSO-d₆) δ 7.72 (s, 1H), 7.51 (s, 1H), 7.24 (s, 1H), 6.48 (s, 1H), 6.30 (s, 2H), 5.42 (s, 2H), 5.27 (d, *J*=3.4 Hz, 2H), 4.28 (d, *J=*8.4 Hz, 1H), 4.13-4.00 (m, 1H), 3.93 (s, 1H), 3.61 (d, *J* = 11.7 Hz, 1H), 3.55 3.40 (m, 5H), 1.87 (td, *J=*14.2*,* 6.8 Hz, 2H), 0.88 (t, *J=7.2* Hz, 3H); LC-MS (M+H)⁺ 492.01 (calculated value 491.17).

### Example 21: 7-(2-(1,3-Dimethoxyisopropyl)amino)ethyl-10,11-methylenedioxycamptothecin (21)

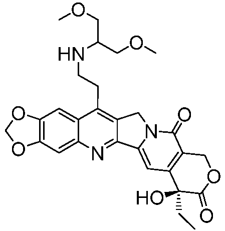

To a reaction flask, 2-amino-1,3-dimethoxypropane (100 mg, 0.84 mmol), hydrochloric acid (0.072 mL, 0.86mmol), 7-methyl-10,11-methylenedioxycamptothecin (50 mg, 0.12 mmol) and DMSO (1.5 mL) were added. The mixture was heated to 120°C under stirring and reacted for 1h, then methyl tert-butyl ether was added. The mixture was filtered and purified by silica gel column chromatography to afford the compound 7-(2-(1,3-dimethoxyisopropyl)amino)ethyl-10,11-methylenedioxycamptothecin (35 mg, yield 54.3%, HPLC 98%); ¹H NMR (500 MHz, DMSO-d₆) δ 8.88 (s, 2H), 7.64 (s, 1H), 7.54 (s, 1H), 7.24 (s, 1H), 6.50 (s, 1H), 6.30 (s, 2H), 5.41 (s, 2H), 5.27 (s, 2H), 3.60 (dt, J=10.7, 6.7 Hz, 5H), 3.41 (s, 2H), 3.32 (s, 5H), 3.28 3.21 (m, 3H), 1.85 (dt, J=14.3, 6.9 Hz, 2H), 0.85 (t, J=7.3 Hz, 3H); LC-MS (M+H)⁺ 538.18 (calculated value 537.21).

### Example 22: 7-(2-(2-Hydroxyethyl)amino)ethyl-10,11-methylenedioxycamptothecin (22)

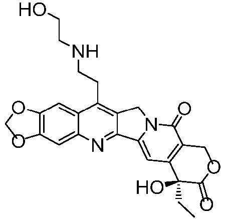

To a reaction flask, ethanolamine (0.5 mL, 8.2 mmol), concentrated hydrochloric acid (0.7 mL, 8.4 mmol), DMSO (10 mL) and 7-methyl-10,11-methylenedioxycamptothecin (0.5 g, 1.2 mmol) were added. The mixture was quickly warmed to 110-120°C and stirred to react for 0.5 hours, then cooled to room temperature, then methyl tert-butyl ether was added. The solid was filtered out and purified by silica gel column chromatography to afford 7-(2-(2-hydroxyethyl)amino)ethyl-10,11-methylenedioxycamptothecin (0.38 g, yield 66%, HPLC 97%); ¹H NMR (500 MHz, DMSO-d₆) δ 8.70 (s, 2H), 7.66 (s, 1H), 7.53 (s, 1H), 7.23 (s, 1H), 6.50 (s, 1H), 6.30 (d, J=1.6 Hz, 2H), 5.42 (s, 2H), 5.27 (s, 2H), 3.82-3.56 (m, 2H), 3.39 (s, 2H), 3.23 (d, J=4.9 Hz, 2H), 3.08 (s, 2H), 1.86 (dt, *J=14.3,* 6.9 Hz, 2H), 0.86 (t, *J*=7.3 Hz, 3H); LC-MC (M+H)⁺ 480.05 (calculated value 479.17).

### Example 23: 7-(2-(2-Trifluoroethyl)amino)ethyl-10,11-methylenedioxycamptothecin (23)

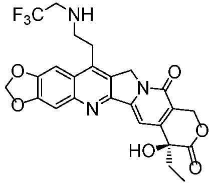

Trifluoroethylamine hydrochloride (100 mg, 0.74 mmol) and 7-methyl-10,11-methylenedioxycamptothecin (100 mg, 0.25 mmol) were dissolved in DMSO (3 mL). The mixture was heated to 120 °C under stirring and reacted for 1h. After cooling, methyl tert-butyl ether was added. The mixture was filtered, and purified by silica gel column chromatography to afford the product7-(2-(2-trifluoroethyl)amino)ethyl-10,11-methylenedioxycamptothecin (49mg, yield 38%, HPLC 99%); ¹H NMR (500 MHz, DMSO-d₆) δ 7.71 (s, 1H), 7.54 (s, 1H), 7.29 (s, 1H), 6.55 (s, 1H), 6.34 (d, *J*=2.0 Hz, 2H), 5.48 (d, *J*=3.0 Hz, 2H), 5.30 (s, 2H), 3.42 (s, 2H), 3.42 (s, 2H), 3.29 (s, 2H), 1.92 (dd, *J=*14.40*,* 7.3 Hz, 2H), 0.94 (t, *J*=7.3 Hz, 4H); LC-MS (M+H)⁺ 518.34 (calculated value 517.15).

### Example 24: 7-(2-(2-Difluoroethyl)amino)ethyl-10,11-methylenedioxycamptothecin (24)

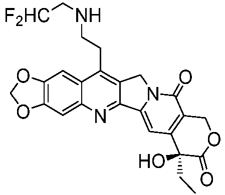

To a reaction flask, difluoroethylamine (1.0 g, 12 mmol), hydrochloric acid (1.4 mL, 16 mmol) and 7-methyl-10,11-methylenedioxycamptothecin (1.0 g, 2.46 mmol) and DMSO (5 mL) were added. The mixture was heated to 120°C under stirring and reacted for 1h, then cooled, then isopropanol was added. The mixture was filtered by suction and purified by silica gel column chromatography to afford the product 7-(2-(2-difluoroethyl)amino)ethyl-10,11-methylenedioxycamptothecin (393 mg, yield 32%, HPLC 95%); ¹H NMR (500 MHz, DMSO-d₆) δ 7.68 (s, 1H), 7.52 (s, 1H), 7.28 (s, 1H), 6.58 (s, 1H), 6.33 (s, 2H), 6.02 (t, J=56.6 Hz, 1H), 5.47 (s, 2H), 5.26 (s, 2H), 3.26 (s, 3H), 2.94 (s, 4H), 1.92 (s, 2H), 0.93 (s, 3H); ¹³C NMR(126 MHz, DMSO) δ 173.06, 157.32, 151.28 , 150.60, 149.74, 149.35, 147.57, 146.86, 141.32, 128.53, 124.98,119.15, 118.45, 117.25, 115.35 ,105.91,105.81, 103.06, 100.05, 96.39, 72.88, 65.73, 49.25, 44.94, 30.79; LC-MS (M+H)⁺ 500.13 (calculated value 499.16).

### Example 25: 7-(2-(Tetrahydropyran-4-yl)amino)ethyl-10,11-methylenedioxycamptothecin (25)

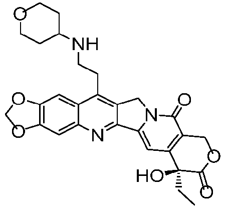

4-Aminotetrahydropyran (0.75 g, 7.4 mmol), concentrated hydrochloric acid (0.7 mL, 7.5 mmol) and DMSO (15 mL) were reacted and warmed to 120°C in an oil bath pan, then 7-methyl-10,11-methylenedioxycamptothecin (0.5 g, 1.2 mmol) was added. The mixture was warmed to 130-140°C, then reacted for 1h, then methanol was added. The mixture filtered by suction and the filtrate was concentrated and purified by silica gel column chromatography to afford the gray solid 7-(2-(tetrahydropyran-4-yl)amino)ethyl-10,11-methylenedioxycamptothecin (0.41 g, yield 63%, HPLC 96%); ¹H NMR (500 MHz, DMSO-d₆) δ 7.59 (s, 1H), 7.47-7.41 (m, 1H), 7.25-7.14 (m, 1H), 6.54 (s, 1H), 6.27 (t, J=9.9 Hz, 2H), 5.77 (s, 1H), 5.44 (d, J=16.5 Hz, 2H), 5.23-5.11 (m, 2H), 3.83 (d, J=9.5 Hz, 2H), 3.23 (t, J=24.9 Hz, 4H), 2.93 (d, J=32.7 Hz, 3H), 1.99-1.72 (m, 4H), 1.33 (s, 2H), 0.96-0.81 (m, 3H); ¹³C NMR(126 MHz, DMSO) δ 173.04, 157.26, 151.30, 150.58, 149.68, 149.42, 147.49, 146.73,146.68, 128.52, 124.79, 118.49, 105.90, 103.14, 99.83, 96.39, 72.82, 66.05, 65.69, 55.41, 53.65, 50.31, 30.66, 8.27; LC-MS (M+H)⁺ 520.11 (calculated value 519.20).

### Example 26: 7-(2-(4-Hydroxycyclohexyl)amino)ethyl-10,11-methylenedioxycamptothecin (26)

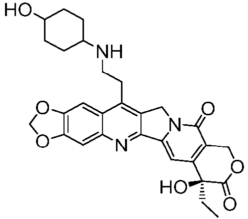

To a reaction flask, 4-aminocyclohexanol (107.05mg, 0.93 mmol), concentrated hydrochloric acid (0.1 mL, 1.2 mmol), DMSO (4 mL) were added. The mixture was reacted and warmed to 100°C in an oil bath pan, then 7-methyl-10,11-methylenedioxycamptothecin (100 mg, 0.25 mmol) was added. The mixture was warmed to 140°C, then reacted for 0.5h, and poured into water. The solid was filtered out and purified by silica gel column chromatography to afford the product 7-(2-(4-hydroxycyclohexyl)amino)ethyl-10,11-methylenedioxycamptothecin (33 mg, yield 28%, HPLC 91%); ¹H NMR (500 MHz, DMSO-d₆) δ 8.47 (s, 1H), 7.67 (s, 1H), 7.57 (s, 1H), 7.27 (s, 1H), 6.33 (s, 2H), 5.44 (s, 2H), 5.35 (s, 2H), 3.81 (s, 2H), 3.25 (s, 2H), 3.13 (d, J=4.6 Hz, 2H), 1.87 (ddd, J=21.5, 12.6, 5.8 Hz, 2H), 1.72 (d, J=20.0 Hz, 6H), 1.46 (d, J=10.8 Hz, 2H), 0.88 (t, J=7.3 Hz, 3H); LC-MS (M+H)⁺ 534.08 (calculated value 533.22).

### Example 27: 7-(2-(4-Methoxycyclohexyl)amino)ethyl-10,11-methylenedioxycamptothecin (27)

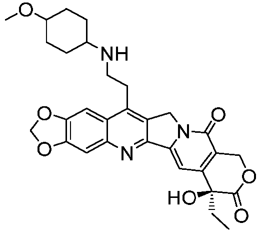

4-Methoxycyclohexylamine (117.7mg, 0.91 mmol), hydrochloric acid (0.08 mL, 0.96 mmol), DMSO (2 mL) and 7-methyl-10,11-methylenedioxycamptothecin (50 mg, 0.12 mmol) were heated to 115-125°C under stirring. The mixture was reacted for 1h, cooled, then methyl tert-butyl ether was added. The solid was filtered out and purified by silica gel column chromatography to afford the compound 7-(2-(4-methoxycyclohexyl)amino)ethyl-10,11-methylenedioxycamptothecin (37 mg, yield 56%, HPLC 98%); ¹H NMR (500 MHz, DMSO-d₆) δ 8.76 (s, 1H), 7.64 (s, 1H), 7.53 (s, 1H), 7.24 (s, 1H), 6.31 (d, J=3.2 Hz, 2H), 5.43 (s, 2H), 5.29 (s, 2H), 3.44 3.36 (m, 2H), 3.22 (dd, *J=11.4,* 4.0 Hz, 5H), 3.12 (d, J=3.9 Hz, 2H), 1.93-1.80 (m, 2H), 1.39 (d, *J=11.4* Hz, 4H), 1.15 (d, J=13.0 Hz, 4H), 0.88 (t, *J=7.2* Hz, 3H); LC-MS (M+H)⁺ 548.35 (calculated value 547.23).

### Example 28: 7-(2-((S)-Hydroxyisopropyl)amino)ethyl-10,11-methylenedioxycamptothecin (28)

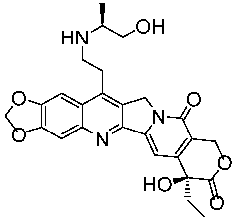

To a reaction flask, (S)-2-amino-1-propanol (110.89 mg, 1.48 mmol), concentrated hydrochloric acid (0.14 mL, 1.68 mmol), DMSO (3 mL) and 7-methyl-10,11-methylenedioxycamptothecin (100 mg, 0.25 mmol) were added respectively. The mixture was heated to 120-130 °C under stirring and reacted for 1h, then cooled to room temperature, then methyl tert-butyl ether was added. The precipitated solid was filtered out and purified by silica gel column chromatography to afford the product 7-(2-((S)-hydroxyisopropyl)amino)ethyl-10,11-methylenedioxycamptothecin (84.94 mg, yield 69%, HPLC 99%); ¹H NMR (500 MHz, DMSO-d₆) δ 8.57 (s, 2H), 7.70 (s, IH), 7.57 (s, 1H), 7.27 (s, 1H), 6.53 (s, 1H), 6.33 (d, *J*=2.0 Hz, 2H), 5.50 (s, 1H), 5.44 (s, 2H), 5.34 (d, *J*=4.6 Hz, 2H), 3.70 (d, *J*=8.5 Hz, 1H), 3.57-3.45 (m, 4H), 3.23 (d, *J=6.5* Hz, 2H), 1.96-177 (m, 2H), 1.21 (d, *J*=6.6 Hz, 3H), 0.88 (t, *J*=7.3 Hz, 3H); LC-MS (M+H)⁺ 494.08 (calculated value 493.18).

### Example 29: 7-(2-((S)-Azidoisopropyl)amino)ethyl-10,11-methylenedioxycamptothecin (29)

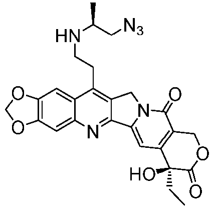

In a 100 mL three-necked flask, compound (S)-2-N-Boc-aminopropanol (1 g, 0.57 mmol) was added under the protection of Ar2, then 10mL of dry DCM was added and stirred to dissolve. After that, the reaction liquid was cooled to 0°C, TEA (0.87g, 0.86mmol) was added, followed by the dropwise addition of MsCl (0.78g, 0.68mmol). The mixture was maintained to stir at 0°C for 2 hours. The reaction liquid was added with water (10mL), extracted with DCM. The organic phase was dried and concentrated to afford the compound (S)-2-N-Boc aminopropanol methanesulfonate (1.4 g, yield 97.2%), which was used directly for the next step without further purification.

(S)-2-N-Boc aminopropanol methanesulfonate (1.4 g, 0.55 mmol) was dissolved in DMF (10mL), then NaN₃ (0.36g, 0.55mmol) was added. The mixture was stirred to react at 40°C overnight. The reaction liquid was added with water (20mL), extracted with EA for three times. After that, the organic phase was combined and washed by water for 3 times, then concentrated and purified by silica gel column chromatography, to afford 1-azido-2-(S)-N-Boc-propylamine (0.8g, yield 72%).

1-Azido-2-(S)-N-Boc-propylamine (0.8 g, 4 mmol) was dissolved in ethyl acetate (2mL), followed by the dropwise addition of a 4N solution of hydrogen chloride in ethyl acetate (5mL 20 mmol). The mixture was stirred for 2 hours, then concentrated to afford 1-azido-2-(S)-isopropylamine (0.5g, yield 92%); ¹H NMR (500 MHz, CDCl₃) δ 4.72 (s, 1H), 3.85 (s, 1H), 3.39 (s, 1H), 3.32 (dd, *J*=12.0, 4.6 Hz, 1H), 1.45 (s, 9H), 1.27-1.04 (m, 3H).

The 1-azido-2-(S)-isopropylamine hydrochloride (147.8 mg, 1.08 mmol) prepared as mentioned above, and 7-methyl-10,11-methylenedioxycamptothecin (100 mg, 0.25 mmol) were dissolved in DMSO (1.5 mL). The mixture was heated to 115-125°C under stirring and reacted for 1 hour, then isopropanol was added. The mixture was filtered and purified by silica gel column chromatography to afford the product 7-(2-((S)-azidoisopropyl)amino)ethyl-10,11-methylenedioxycamptothecin (57 mg, yield 44%, HPLC 93%); ¹H NMR (600 MHz, DMSO-d₆) δ 7.68 (s, 1H), 7.55 (m, 1H), 7.26 (s, 1H), 6.52 (s, 1H), 6.46 (s, 2H), 5.45 (d, *J*=16.7 Hz, 2H), 5.36-5.25 (m, 2H), 4.80-4.60 (m, 1H), 3.85-3.8 (m, z, 2H)3.52-3.38 (m, 4H), 1.86 (m, *J*=28.9, 14.5, 7.3 Hz, 2H), 1.57-1.46 (m, 3H), 0.87 (t, *J*=7.3 Hz, 3H); LC-MS (M+H)+ 519.02 (calculated value 518.19).

### Example 30: 7-(2-(N-Hydroxyacetyl((S)-methoxyisopropyl))amino)ethyl-10,11-methylenedioxycamptothecin (30)

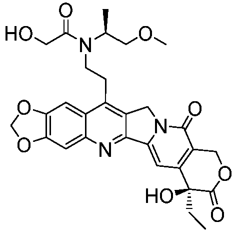

Glycolic acid (7 mg, 0.09 mmol) was added to DMF (1 mL), then HATU (25mg, 0.065 mmol) and DIPEA (7.0 µL) were added sequentially under ice-bath condition. After stirring for 30min under ice-bath condition, 7-(2-((S)-2-methoxyisopropyl)amino)ethyl-10,11-methylenedioxycamptothecin (10 mg, 0.02 mmol, Example 19) was added. The mixture was slowly warmed to room temperature, then stirred for 18h, and prepared using 0.1% TFA (A) and acetonitrile (B): A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the product 7-(2-(N-hydroxyacetyl((S)-methoxyisopropyl))amino)ethyl-10,11-methylenedioxycamptothecin (6 mg, yield 54%, HPLC 95%); LC-MS (M+H)+ 566.58 (calculated value 565.21).

### Example 31: 7-(2-(N-Hydroxyacetyl(2-azidoethyl))amino)ethyl-10,11-methylenedioxycamptothecin (31)

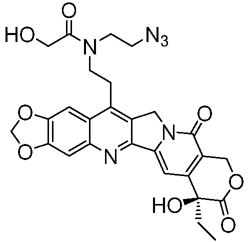

Glycolic acid (7 mg, 0.09 mmol) was added to DMF (1 mL), then HATU (25mg, 0.065 mmol) and DIPEA (7.0 µL) were added sequentially under ice-bath condition. After stirring for 30min under ice-bath condition, 7-(2-(2-azidoethyl)amino)ethyl-10,11-methylenedioxycamptothecin (10 mg, 0.02 mmol, Example 16) was added. The mixture was slowly warmed to room temperature, then stirred for 18h, and prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the product 7-(2-(N-hydroxyacetyl(2-azidoethyl))amino)ethyl-10,11-methylenedioxycamptothecin (6 mg, yield 58%, HPLC 95%); ¹H NMR (500 MHz, DMSO-d₆) δ 7.67 (s, 1H), 7.55 (s, 1H), 7.25 (s, 1H), 6.51 (s, 1H), 6.32 (s, 2H), 5.43 (s, 2H), 5.30 (s, 2H), 3.91 (s, 2H), 3.79 3.68 (m, 2H), 3.26 (s, 2H), 3.20 (s, 2H), 2.62 (d, *J*=4.6 Hz, 1H), 2.54 (d, *J*=7.6 Hz, 1H), 1.86 (ddd, J=21.5, 14.2, 7.0 Hz, 2H), 0.88 (t, *J*=7.3 Hz, 3H); LC-MS (M+H)⁺ 563.54 (calculated value 562.18).

### Example 32: 7-(2-(N-Hydroxyacetyl(2-methoxyethyl))amino)ethyl-10,11-methylenedioxycamptothecin (32)

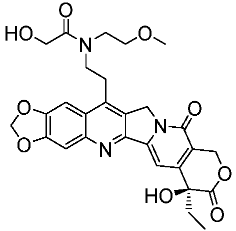

Glycolic acid (7 mg, 0.09 mmol) was added to DMF (1 mL), then HATU (25mg, 0.065 mmol) and DIPEA (7.0 µL) were added sequentially under ice-bath condition. After stirring for 30min under ice-bath condition, 7-(2-(2-methoxyethyl)amino)ethyl-10,11-methylenedioxycamptothecin (10 mg, 0.02 mmol, Example 17) was added. The mixture was slowly warmed to room temperature, then stirred for 18h, and prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the product 7-(2-(N-hydroxyacetyl(2-methoxyethyl))amino)ethyl-10,11-methylenedioxycamptothecin (6 mg, yield 60%, HPLC 95%); ¹H NMR (500 MHz, DMSO-d₆) δ 7.84 (s, 1H), 7.48 (s, 1H), 7.22-7.16 (m, 1H), 6.29 (d, J=2.8 Hz, 2H), 5.42 (s, 2H), 5.24 (s, 3H), 3.56-3.45 (m, 8H), 3.37-3.21 (m, 5H), 1.99-1.77 (m, 2H), 0.88 (t, *J=7.2* Hz, 3H); LC-MS (M+H)⁺ 552.55 (calculated value 551.19).

### Example 33: 7-(2-(N-Hydroxyacetyl(2-difluoroethyl))amino)ethyl-10,11-methylenedioxycamptothecin (33)

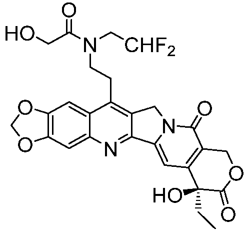

Glycolic acid (7 mg, 0.09 mmol) was added to DMF (1 mL), then HATU (23 mg, 0.06 mmol) and DIPEA (7.0 µL) were added sequentially under ice-bath condition. After stirring for 30min under ice-bath condition, 7-(2-(2-difluoroethyl)amino)ethyl-10,11-methylenedioxycamptothecin (10 mg, 0.019 mmol, Example 24) was added. The mixture was slowly warmed to room temperature, then stirred for 18h, and prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the compound 7-(2-(N-hydroxyacetyl(2-difluoroethyl))amino)ethyl-10,11-methylenedioxycamptothecin (6 mg, yield 54%, HPLC 95%); ¹H NMR (500 MHz, DMSO-d₆) δ 7.91 (s, 1H), 7.51 (d, *J*=5.2 Hz, 1H), 7.24 (d, *J*=3.2 Hz, 1H), 6.30 (s, 2H), 5.42 (s, 2H), 5.30 (d, *J*=6.1 Hz, 2H), 4.16 (d, *J*=31.8 Hz, 2H), 3.92 (t, *J*=12.6 Hz, 2H), 3.66-3.54 (m, 2H), 3.44 (s, 1H), 3.38-3.24 (m, 2H), 1.87 (dt, *J=14.2,* 6.9 Hz, 2H), 0.88 (t, *J*=7.3 Hz, 3H); LC-MS (M+H)⁺ 557.55 (calculated value 557.16).

### Example 34: 7-(2-(N-(R)-Tetrahydrofuran-3-ylamino))ethyl-10,11-methylenedioxycamptothecin (34)

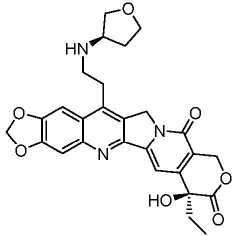

To a reaction flask, (R)-3-aminotetrahydrofuran (64.31mg, 0.738 mmol),concentrated hydrochloric acid(0.05mL, 0.6 mmol), DMSO (1. 5mL) and 7-methyl-10,11-methylenedioxycamptothecin (50mg, 0.12 mmol) were added respectively. The mixture was stirred to heated to 120-130 °C and reacted for 1h, then cooled to room temperature, then methyl tert-butyl ether was added. The precipitated solid was filtered out and purified by silica gel column chromatography to afford the product 7-(2-(N-(R)-tetrahydrofuran-3-ylamino))ethyl-10,11-methylenedioxycamptothecin (16.2 mg, yield 32.3%, HPLC 96.2%); ¹H NMR (500 MHz, DMSO-d₆) δ 7.61 (s, 1H), 7.47 (s, 1H), 7.22 (s, 1H), 6.50 (s, 1H), 6.28 (s, 2H), 5.42 (s, 2H), 5.21 (s, 2H), 4.21-3.53 (m, 4H), 3.21 (t, *J*=7.6 Hz, 2H), 2.84 (q, *J*=8.7, 8.1Hz, 2H), 1.90 (ddt, *J*=31.9, 16.2, 7.1 Hz, 3H), 1.63 (dd, *J*=12.3, 6.1 Hz, 1H), 0.88 (t, *J*=7.3 Hz, 3H); ¹³C NMR (126 MHz, DMSO) δ 173.0, 157.3, 151.3, 150.6, 149.7, 149.4, 147. 5, 146.8, 141.2, 128.6, 124.9, 118.4, 105.9, 103.1, 99.9, 96.4, 72.9, 66.9, 65.7, 58.4, 55.4, 50.4, 47.6, 32.6, 30.7, 30.6, 8.3; LC-MS (M+H)⁺ 506.34 (calculated value 505.18).

### Example 35: 7-(2-(N-(S)-Tetrahydrofuran-3-ylamino))ethyl-10,11-methylenedioxycamptothecin (35)

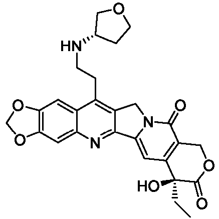

To a reaction flask, (S)-3-aminotetrahydrofuran (64.31 mg, 0.738 mmol),concentrated hydrochloric acid(0.05 mL, 0.6 mmol), DMSO (1.5 mL) and 7-methyl-10,11-methylenedioxycamptothecin (50 mg, 0.12 mmol) were added respectively. The mixture was stirred to heated to 120-130 °C and reacted for 1h, then cooled to room temperature, then methyl tert-butyl ether was added. The precipitated solid was filtered out and purified by silica gel column chromatography to afford the product 7-(2-(N-(S)-tetrahydrofuran-3-ylamino))ethyl-10,11-methylenedioxycamptothecin (18 mg,yield 33%, HPLC 95.9%); ¹H NMR (500 MHz, DMSO-d₆) δ 7.61 (d, *J*=3.4 Hz, 1H), 7.47 (d, *J*=3.9 Hz, 1H), 7.22 (d, *J*=4.2 Hz,1H), 6.50 (s, 1H), 6.29 (d, *J*=4.0 Hz, 2H), 5.42 (d, *J=*4.0 Hz, 2H), 5.21 (d, *J*=3.2 Hz, 2H), 3.87-3.58 (m, 4H), 3.21 (s, 2H), 2.85 (td, *J*=11.2, 10.7, 5.8 Hz, 2H), 1.90 (ddt, *J*=29.9, 10.7, 6.2 Hz, 3H), 1.68-1.60 (m, 1H), 1.08-0.76 (m, 3H); ¹³C NMR (126 MHz, DMSO) δ 173.0, 157.3, 151.3, 150.6, 149.7, 149.4, 147. 6, 146.9, 141.2, 128.6, 124.9, 118.5, 105.9, 103.1, 100.0, 96.4, 72.9, 72.7, 66.9, 65.7, 58.4, 50.4, 47.6, 32.6, 30.7, 30.6, 8.2; LC-MS (M+H)⁺ 506.31 (calculated value 505.18).

### Example 36: 7-(2-Difluoroacetylamino)ethyl-10,11-methylenedioxycamptothecin (36)

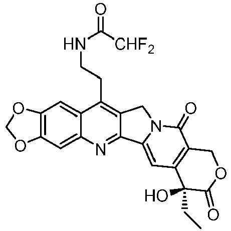

160 mg of ammonium chloride and 6 drops of hydrochloric acid were added to DMSO (9mL). The mixture was heated to 120°C in an oil bath pan, then 7-methyl-10,11-methylenedioxycamptothecin (200mg, 0.49 mmol) was added. The mixture was warmed to 130°C and reacted for 1h, then cooled to room temperature. The mixture was added with methanol for trituration, filtered by suction, concentrated to remove part of the DMSO, and prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the product 7-aminoethyl-10,11-methylenedioxycamptothecin (99 mg, yield 46%, HPLC 96%); ¹H NMR (500 MHz, DMSO-d₆) δ 7.99 (s, 2H), 7.70 (s, 1H), 7.59 (s, 1H), 7.30 (s, 1H), 6.37 (s, 2H), 5.49 (s, 2H), 5.32 (s, 2H), 3.67-3.49 (m, 2H), 3.18 (s, 2H), 1.93 (dd, *J=10.5,* 7.3 Hz, 2H), 0.94 (t, *J*=7.3 Hz, 3H); ¹³C NMR(126 MHz, DMSO) δ 173.01, 157.29, 151.46, 150.63, 150.01, 149.76, 147.65, 146.66, 137.76, 129.10, 124.77, 118.63, 106.14, 103.25, 99.61, 96.48, 72.88, 65.69, 50.31, 38.50, 30.70, 27.97, 8.23; LC-MS (M+H)⁺ 436.41 (calculated value 435.14).

7-Aminoethyl-10,11-methylenedioxycamptothecin (20 mg, 0.046 mmol) was added to a reaction flask, DMF (5 mL) was added, difluoroacetic acid (8.8 mg, 0.092 mmol) was added, then HATU (35 mg, 0.092 mmol) and DIPEA (12 mg, 0.092 mmol) were added. The mixture was reacted for one hour at room temperature. The reaction was quenched by dropwise addition of 0.5mL water. The reaction liquid was prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the target product 7-(2-difluoroacetylamino)ethyl-10,11-methylenedioxycamptothecin (14.6 mg, yield 62%, HPLC 98%); ¹H NMR (500 MHz, DMSO-d₆) δ 9.00 (s, 1H), 7.67 (s, 1H), 7.51 (s, 1H), 7.24 (s, 1H), 6.30 (s, 2H), 5.43 (s, 2H), 5.24 (s, 2H), 3.51 (dd, J=13.6, 6.8 Hz, 2H), 3.31-3.23 (m, 2H), 2.07-1.75 (m, 2H), 0.90-0.80 (m, 3H); ¹³C NMR(126 MHz, DMSO) δ 173.02, 157.30, 151.39, 150.61, 149.83, 149.55, 147.63, 146.77, 139.56, 128.71, 125.06, 118.54, 109.02, 106.04, 103.14, 99.81, 96.42, 72.88, 65.71, 53.93, 50.23, 42.21, 38.28, 30.67, 13.04; LC-MS (M+H)⁺ 514.25 (calculated value 513.13).

### Example 37: 7-(2-Azidoacetylamino)ethyl-10,11-methylenedioxycamptothecin (37)

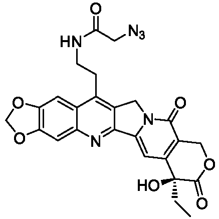

7-Aminoethyl-10,11-methylenedioxycamptothecin (15 mg, 0.035 mmol) was added to a reaction flask, DMF (5 mL) was added, azidoacetic acid (7.2 mg, 0.071 mmol) was added, then HATU (35mg, 0.092mmol) and DIPEA (12 mg, 0.092 mmol) were added. The mixture was reacted for one hour at room temperature. The reaction was quenched by dropwise addition of 0.5mL water. The reaction liquid was prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the target product 7-(2-azidoacetylamino)ethyl-10,11-methylenedioxycamptothecin (13.1 mg, yield 71%, HPLC 95%); ¹H NMR (500 MHz, DMSO-d₆) δ 8.33 (s, 1H), 7.69 (s, 1H), 7.51 (s, 1H), 7.23 (s, 1H), 6.49 (s, 1H), 6.30 (s, 2H), 5.42 (s, 2H), 5.24 (s, 2H), 3.80 (s, 2H), 3.46 (d, *J*=6.3 Hz, 2H), 3.25 (s, 2H), 1.86 (dt, *J=18.9,* 6.9 Hz, 2H), 0.87 (d, *J*=7.3 Hz, 3H); LC-MS (M+H)⁺ 519.29 (calculated value 518.15).

### Example 38: 7-(2-Methanesulfonamido)ethyl-10,11-methylenedioxycamptothecin (38)

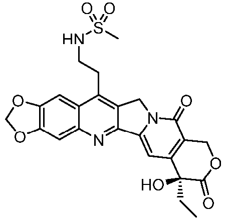

7-Aminoethyl-10,11-methylenedioxycamptothecin (15 mg, 0.034 mmol) was added to reaction flask, DMF (5 mL) was added, methanesulfonyl chloride (5 mg, 0.043 mmol) was added, then DIPEA (47.5 mg, 0.368 mmol) was added. The mixture was reacted for one hour at room temperature. The reaction was quenched by dropwise addition of 0.5mL water. The reaction liquid was prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the product 7-(2-methanesulfonamido)ethyl-10,11-methylenedioxycamptothecin (14.2 mg, yield 80%, HPLC 96%); ¹H NMR (500 MHz, DMSO-d₆) δ 7.56 (s, 1H), 7.48 (s, 1H), 7.21 (d, J=8.4 Hz, 2H), 6.28 (d, J=2.1 Hz, 3H), 5.42 (d, J=3.1 Hz, 2H), 5.19 (s, 2H), 3.27 (s, 4H), 2.84 (s, 3H), 1.87 (dt, *J=14.5,* 7.0 Hz, 2H), 0.89 (t, J=7.3 Hz, 3H); LC-MS (M+H)⁺ 514.25 (calculated value 513.12).

### Example 39: 7-(2-Cyclopropylsulfonamido)ethyl-10,11-methylenedioxycamptothecin (39)

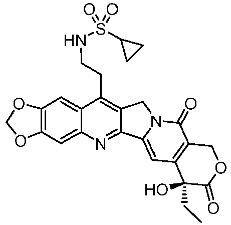

7-Aminoethyl-10,11-methylenedioxycamptothecin (20 mg, 0.046 mmol) was added to a reaction flask, DMF (5 mL) was added, cyclopropylsulfonyl chloride (12.9 mg, 0.092 mmol) was added, then DIPEA (47.5 mg, 0.368 mmol) was added. The mixture was reacted for one hour at room temperature. The reaction was quenched by dropwise addition of 0.5mL water. The reaction liquid was prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the product 7-(2-cyclopropylsulfonamido)ethyl-10,11-methylenedioxycamptothecin (19.4 mg, yield 78%, HPLC 98%); ¹H NMR (500 MHz, DMSO-d₆) δ 7.60 (s, IH), 7.50 (s, IH), 7.28 (s, IH), 7.23 (s, IH), 6.49 (s, IH), 6.29 (s, 2H), 5.42 (s, 2H), 5.25 (s, 2H), 3.35 (s,2H), 3.29-3.20 (m, 2H), 1.87 (td, *J=*14.2*,* 7.0 Hz, 2H), 1.24 (ddd, *J*=20.4, 13.3, 6.6 Hz, 2H), 0.94-0.79 (m, 6H); LC-MS (M+H)⁺ 540.26 (calculated value 539.14).

### Example 40: 7-(2-(Pyrazol-5-ylamino))ethyl-10,11-methylenedioxycamptothecin (40)

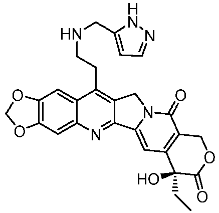

1H-3-Pyrazolemethanamine (71.69 mg, 0.74 mmol) and hydrochloric acid (72 µL) were added to DMSO (1.5 mL). The mixture was warmed to 110°C in an oil bath pan, then 7-methyl-10,11-ethylenedioxycamptothecin (50 mg, 0.12 mmol) was added. The mixture was warmed to 135 °C and continued to react for 1h. After cooling, methanol was added for trituration. The mixture was filtered by suction and prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the target product 7-(2-(pyrazol-5-ylamino))ethyl-10,11-methylenedioxycamptothecin (20.8 mg, yield 33.6%, HPLC 98%); LC-MS (M+H)⁺ 516.35 (calculated value 515.18).

### Example 41: 7-(2-(1,2,4-Triazol-5-ylamino))ethyl-10,11-methylenedioxycamptothecin (41)

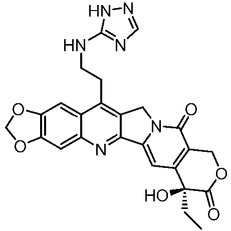

1H-1,2,4-Triazol-3-amine (62.07 mg, 0.74 mmol) and hydrochloric acid (72 µL) were added to DMSO (1.5 mL). The mixture was warmed to 120°C under stirring, then 7-methyl-10,11-ethylenedioxycamptothecin (50 mg, 0.12 mmol) was added. The mixture was warmed to 140°C and continued to react for 1h, then cooled to room temperature, then methanol was added. The mixture was filtered by suction, concentrated and prepared using 0.1% TFA (A) and acetonitrile (B), 0-4min: A: 90%-75%, B: 10%-25%; 4-25min: A: 75%-60%, B: 25%-40%; 25-35min: A: 60%-50%, B-40%-50%; 35-60min: A: 50%-10%, B: 50%-90%, to afford the target product 7-(2-(1,2,4-triazol-5-ylamino))ethyl-10,11-methylenedioxycamptothecin (31.4 mg, yield 52%, HPLC 96%); LC-MS (M+H)⁺ 503.30 (calculated value 502.16).

### Example 42: 7-(2-(2-Methoxyethyl)amino)ethyl-10,11-ethylenedioxycamptothecin (42)

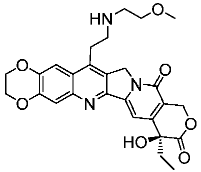

To a single-neck flask containing anhydrous 1,2-dichloroethane (160 mL), a 1M solution of BCl₃ in dichloromethane (32 mL, 32 mmol BCl₃ dissolved in dichloromethane) was added. The reaction flask was cooled to 0°C, then 3.4-ethyldioxyaniline (5 g, 33.08 mmol) was added. The mixture was reacted at 0°C for 10min, then acetonitrile (16.40g, 409.54mmol) and aluminum trichloride (7g, 52.5mmol) were added. The mixture was slowly warmed to room temperature and stirred for 10min, then warmed to 80°C and stirred for 12h. The reaction liquid was poured into ice water, and a 1M solution of HCl was added to adjust the pH=2. The mixture was extracted with dichloromethane, dried over Na₂SO₄ and purified by silica gel column chromatography to afford 6-amino-3,4-ethyldioxyacetophenone (2 g, yield 31.3%, HPLC 95%); LC-MS (M+H)⁺ 194.17 (calculated value 193.07)..

6-Amino-3,4-ethyldioxyacetophenone (0.5 g, 0.26 mmol) was dissolved in anhydrous toluene (30 mL), then "tricyclic ketone" (0.68 g, 0.26 mmol) and PPTS (0.065g, 0.026mmol) were added. The reaction liquid was heated to 115°C and stirred for 12 hours. The solvent was removed by concentrating under reduced pressure, and 5 mL of methanol was added. The mixture was filtered, and the filter cake was dried to afford 7-methyl-10,11-ethylenedioxycamptothecin (1.0 g, yield 91%, HPLC 95%); ¹H NMR (500 MHz, DMSO-d₆) δ 7.55 (d, *J=19.7* Hz, 2H), 7.25 (s, 1H), 6.48 (s, 1H), 5.42 (s, 2H), 5.19 (s, 2H), 4.44 (s, 4H), 2.66 (s, 3H), 1.86 (s, 2H), 0.88 (s, 3H); LC-MS (M+H)⁺ 421.15 (calculated value 420.13).

2-Methoxyethylamine (33 mg, 0.44 mmol), hydrochloric acid (0.035 mL, 0.42 mmol), DMSO (1 mL) and 7-methyl-10,11-ethylenedioxycamptothecin (25 mg, 0.06 mmol) were warmed to 120-130°C under stirring, then reacted for 30 minutes, then cooled to room temperature, then isopropanol was added. The mixture was filtered and purified by silica gel column chromatography to afford the product 7-(2-(2-methoxyethyl)amino)ethyl-10,11-ethylenedioxycamptothecin (17 mg, yield 55.7%, HPLC 97%); ¹H NMR (500 MHz, DMSO-d₆) δ 8.68 (s, 2H), 7.73 (s, 1H), 7.60 (s, 1H), 7.26 (s, 1H), 6.52 (s, 1H), 5.44 (s, 2H), 5.31 (s, 2H), 4.46 (s, 4H), 3.67-3.55 (m, 2H), 3.43 (s, 3H), 3.27-3.15 (m, 5H), 1.97-1.77 (m, 2H), 0.88 (t, J=7.3 Hz, 3H); LC-MS (M+H)⁺ 508.18 (calculated value 507.20).

### Example 43: 7-(2-(Tetrahydropyran-4-yl)amino)ethyl-10,11-ethylenedioxycamptothecin (43)

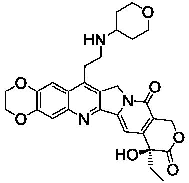

4-Aminotetrahydropyran (72 mg, 0.71 mmol) and hydrochloric acid (0.06 mL, 0.72 mmol) were added to DMSO (1.5.mL). The mixture was warmed to 120°C under stirring, then 7-methyl-10,11-ethylenedioxycamptothecin (50 mg, 0.12 mmol) was added. The mixture was warmed to 130°C and continued to react for 30 minutes, then cooled to room temperature, then methyl tert-butyl ether was added. The mixture was filtered and purified by silica gel column chromatography to afford the compound 7-(2-(tetrahydropyran-4-yl)amino)ethyl-10,11-ethylenedioxycamptothecin (37 mg, yield 57.8%, HPLC 95%); LC-MS (M+H)+ 534.19 (calculated value 533.22).

### Example 44: 7-(2-(4-Methoxycyclohexyl)amino)ethyl-10,11-ethylenedioxycamptothecin (44)

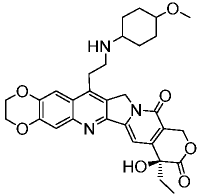

To a reaction flask, 4-methoxycyclohexylamine (52 mg, 0.45 mmol), hydrochloric acid (0.035 mL, 0.42 mmol), DMSO (1.5 mL) and 7-methyl-10,11-ethylenedioxycamptothecin (50 mg, 0.12 mmol) were added. The mixture was warmed to 120-130°C under stirring and reacted for 45 minutes, then isopropanol was added. The mixture was filtered and purified by silica gel column chromatography to afford the compound 7-(2-(4-methoxycyclohexyl)amino)ethyl-10,11-ethylenedioxycamptothecin (34 mg, yield 50.5%, HPLC 97%); ¹H NMR (500 MHz, DMSO-d₆) δ 8.56 (s, 2H), 7.69 (s, 1H), 7.60 (s, 1H), 7.28 (s, 1H), 5.44 (s, 2H), 5.32 (s, 2H), 4.46 (s, 4H), 3.31 (s, 2H), 3.24 (s, 5H), 3.12 (d, *J=10.1* Hz, 2H), 2.06 (d, J=10.3 Hz, 4H), 1.96-1.81 (m, 2H), 1.37 (dd, J=23.1, 11.7 Hz, 2H), 1.16 (dd, *J=22.9,* 10.6 Hz, 2H), 0.88 (t, J=7.3 Hz, 3H); LC-MS (M+H)⁺ 562.24 (calculated value 561.25).

### Example 45: 7-(2-((S)-Methoxyisopropyl)amino)ethyl-10,11-ethylenedioxycamptothecin (45)

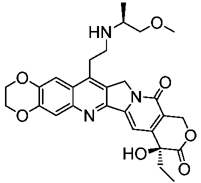

(S)-1-Methoxy-2-propylamine (47.5 mg, 0.53 mmol) and hydrochloric acid (0.04 mL, 0.48 mmol) were added to a reaction flask containing DMSO (1mL). The mixture was warmed to 110°C under stirring, then 7-methyl-10,11-ethylenedioxycamptothecin (50 mg, 0.12 mmol) was added. The mixture was warmed to 130°C, then reacted for 30 minutes, then cooled to room temperature, then isopropanol was added. The mixture was filtered and purified by silica gel column chromatography to afford the product 7-(2-((S)-methoxyisopropyl)amino)ethyl-10,11-ethylenedioxycamptothecin (34.6 mg, yield 55.3%, HPLC 98%); ¹H NMR (500 MHz, DMSO-d₆) δ 8.64 (s, 1H), 7.70 (s, 1H), 7.58 (s, 1H), 7.25 (s, 1H), 5.41 (s, 1H), 5.29 (d, *J=4.7* Hz, 1H), 4.44 (s, 4H), 3.59-3.54 (m, 2H), 3.44 (dd, *J*=10.3, 5.7 Hz, 4H), 3.23 (d, *J=*19.3 Hz, 4H), 1.92-1.79 (m, 2H), 1.21 (d, *J*=6.5 Hz, 3H), 0.85 (t, *J*=7.3 Hz, 3H); LC-MS (M+H)⁺ 522.16 (calculated value 521.22).

### Example 46: Tumor cell growth inhibitory activity

Human esophageal cancer cells OE33 (human breast adenocarcinoma cells SK-BR-3, or human gastric cancer cells NCI-N87) was cultured in RPMI1640 (Cellmax) supplemented with 10% fetal bovine serum (Cellmax). Cells in the exponential growth phase were diluted to a concentration of 1×10⁵cells/mL by culture medium and added to 96-well cell culture plates at 100µL per well, then returned back and incubated overnight in a 37°C incubator with 5% CO₂. On the following day, the compounds were diluted to concentrations of 10000 nM, 2000 nM, 400 nM, 80 nM, 16 nM, 3.2 nM, 0.64 nM, and 0.13 nM by culture medium and the diluted compounds were added to the 96-well cell culture plates at 2µL per well, with three replicate wells for each concentration. Negative control and blank control groups without addition of compounds were added with 2µL of the dilution liquid per well. After the addition of the compounds, the plates were returned back to the 37°C incubator with 5% CO₂ to continue the incubation for 72 h. After the incubation, the cell culture plates were removed, and the culture medium was aspirated using a pipette. Then, 100µL of culture medium containing 10% CCK-8 was added to each well, and the plates were incubated at 37°C for 3 h. After the incubation, the plates were removed, kept in the dark, and placed in an ELISA plate. Absorbance was measured at 630nm as the reference wavelength and 450nm as the test wavelength. The IC₅₀ values (Table 2) were calculated using the four-parameter logistic regression method in GraphPad.

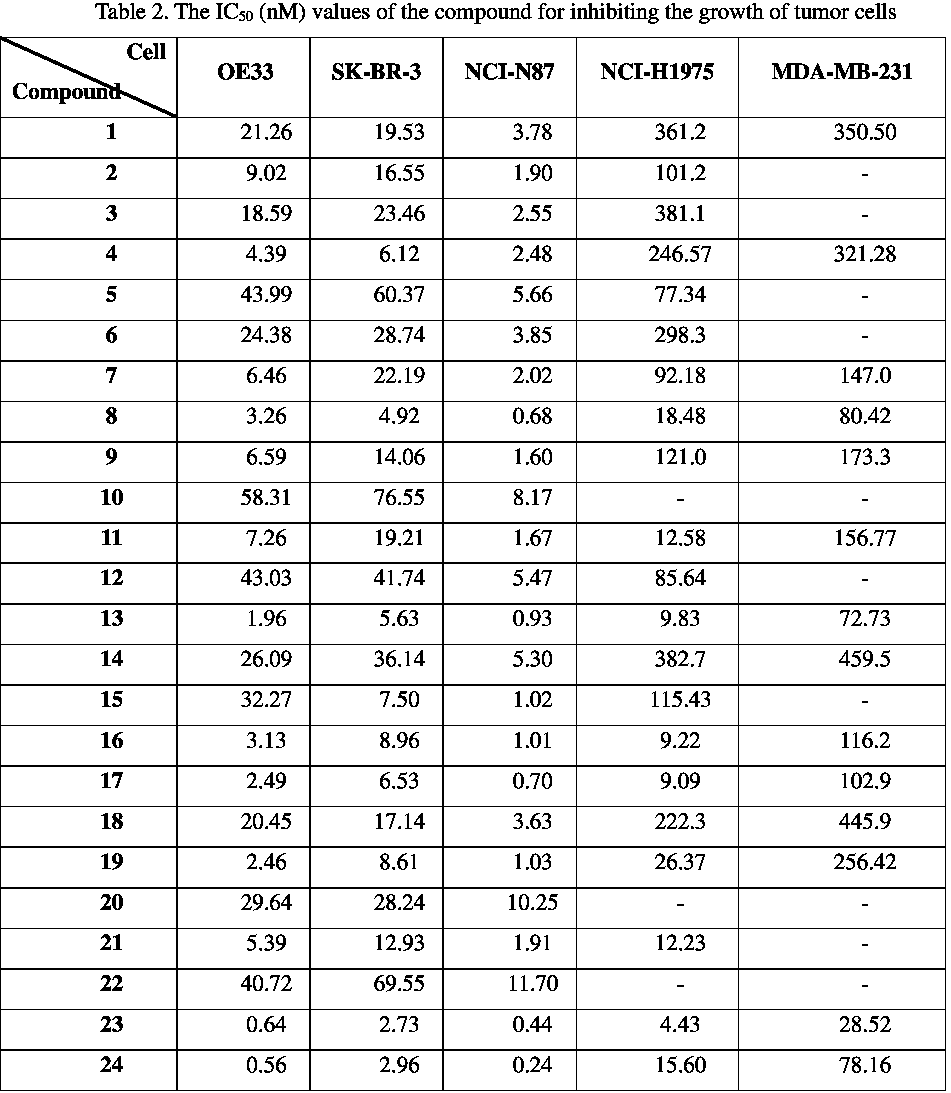

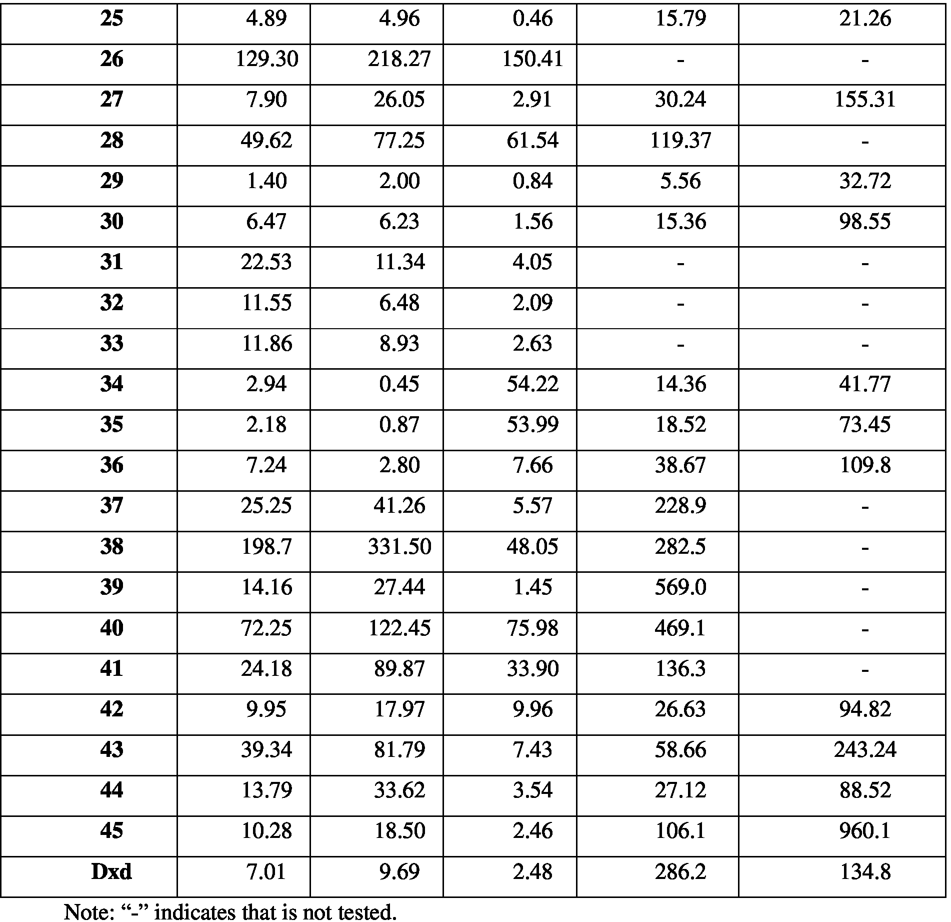

The above description is merely examples of the present disclosure and is not intended to limit the protection scope of the present disclosure. Any modifications, equivalent substitutions, and improvements made within the spirit and principle of the present disclosure shall all be included within the protection scope of the present disclosure.

## Claims

1. A compound represented by Formula 1 or a pharmaceutically acceptable salt, a stereoisomer, or a prodrug thereof, 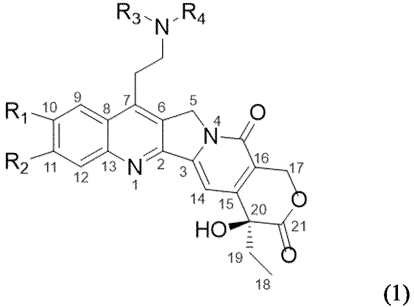
wherein, each of R₁ and R₂ independently represents halogen, hydroxy, alkyl, alkoxy, or R₁ and R₂ collectively form a methylenedioxy bridge or an ethylenedioxy bridge;
each of R₃ and R₄ independently represents hydrogen, hydroxy, alkyl, alkoxy, cycloalkyl, heterocycloalkyl, aryl, heteroaryl, acyl, sulfonyl, or R₃, R₄ and the nitrogen atom to which they connect collectively form a heterocycloalkyl or heteroaryl, the alkyl, alkoxy, cycloalkyl, alkylacyl, sulfonyl, heterocycloalkyl, aryl, or heteroaryl is optionally substituted by R,
R is selected from halogen, hydroxy, alkyl, alkoxy, cycloalkyl, azide or 5 to 7 membered heteroaryl.

2. The compound or pharmaceutically acceptable salt, stereoisomer, or prodrug thereof according to claim 1, wherein, each of R₁ and R₂ independently represents halogen, hydroxy, C₁-C₆ alkyl, C₁-C₆ alkoxy, or R₁ and R₂ collectively form a methylenedioxy bridge or an ethylenedioxy bridge;
preferably, each of R₁ and R₂ independently represents halogen, methyl, or R₁ and R₂ collectively form a methylenedioxy bridge or an ethylenedioxy bridge,
preferably, each of R₁ and R₂ independently represents methyl, F, Cl, Br, I, or R₁ and R₂ collectively form a methylenedioxy bridge or an ethylenedioxy bridge;
preferably, each of R₁ and R₂ independently represents methyl, F, or R₁ and R₂ collectively form a methylenedioxy bridge or an ethylenedioxy bridge.

3. The compound or pharmaceutically acceptable salt, stereoisomer, or prodrug thereof according to claim 1 or 2, wherein, each of R₃ and R₄ independently represents hydrogen, C₁-C₆ alkyl, C₁-C₆ alkoxy, C₃-C₆ cycloalkyl, 4 to 8 membered heterocycloalkyl, 5 to 12 membered aryl, 5 to 12 membered heteroaryl, C₁-C₆ alkyl-acyl, C₁-C₆ alkyl-sulfonyl, C₃-C₆ cycloalkyl-sulfonyl, or R₃, R₄ and the nitrogen atom to which they connect collectively form a 4 to 8 membered heterocycloalkyl, the C₁-C₆ alkyl, C₁-C₆ alkoxy, C₃-C₆ cycloalkyl, 4 to 8 membered heterocycloalkyl, 5 to 12 membered aryl, 5 to 12 membered heteroaryl, C₁-C₆ alkyl-acyl, C₁-C₆ alkyl-sulfonyl, or C₃-C₆ cycloalkyl-sulfonyl is optionally substituted by R;
preferably, each of R₃ and R₄ independently represents C₁-C₆ alkyl, C₃-C₆ cycloalkyl, 5 to 6 membered heterocycloalkyl, 5 to 6 membered heteroaryl, C₁-C₃ alkyl-acyl, C₁-C₃ alkyl-sulfonyl, C₃-C₆ cycloalkyl-sulfonyl, or R₃, R₄ and the nitrogen atom to which they connect collectively form a 5 to 6 membered heterocycloalkyl, the C₁-C₆ alkyl, C₃-C₆ cycloalkyl, 5 to 6 membered heterocycloalkyl, 5 to 6 membered heteroaryl, C₁-C₃ alkyl-acyl, C₁-C₃ alkyl-sulfonyl, or C₃-C₆ cycloalkyl-sulfonyl is optionally substituted by R;
preferably, each of R₃ and R₄ independently represents C₁-C₆ alkyl, C₃-C₆ cycloalkyl, 5 to 6 membered heterocycloalkyl, 5 to 6 membered heteroaryl, C₁-C₃ alkyl-acyl, C₁-C₃ alkyl-sulfonyl, C₃-C₆ cycloalkyl-sulfonyl, or R₃, R₄ and the nitrogen atom to which they connect collectively form a 5 to 6 membered heterocycloalkyl, the C₁-C₆ alkyl, C₃-C₆ cycloalkyl, 5 to 6 membered heterocycloalkyl, 5 to 6 membered heteroaryl, C₁-C₃ alkyl-acyl, C₁-C₃ alkyl-sulfonyl, or C₃-C₆ cycloalkyl-sulfonyl is optionally substituted by R, or R₃, R₄ and the nitrogen atom to which they connect collectively form a 5 to 6 membered heterocycloalkyl, the heterocycloalkyl, or heteroaryl contains 1-3 heteroatoms independently selected from N, O;
preferably, R₃ and R₄ are each independently selected from the following groups: 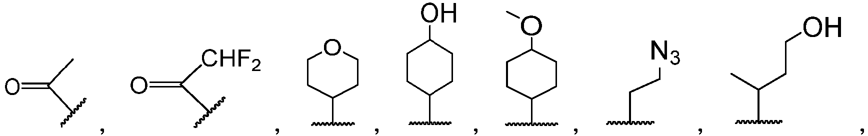 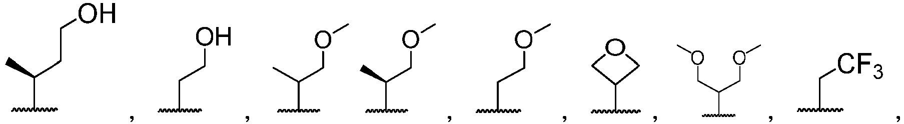 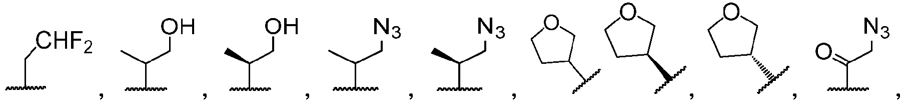 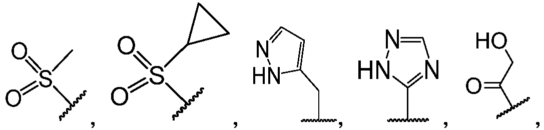
or R₃, R₄ and the nitrogen atom to which they connect collectively form pyrrolidinyl, piperidinyl, piperazinyl,
preferably, the -NR₃R₄ is each independently or collectively the following structure: 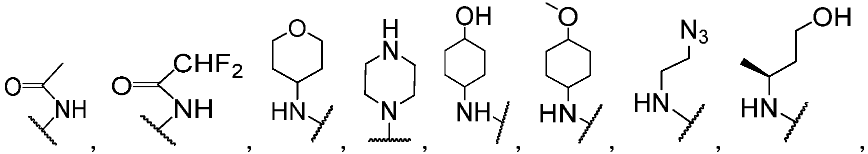 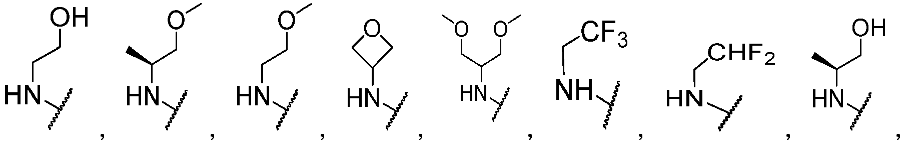 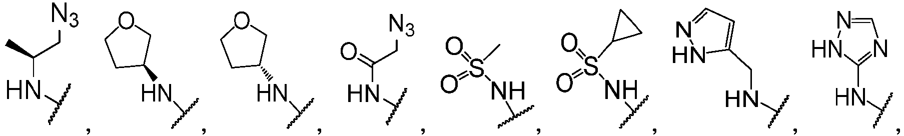 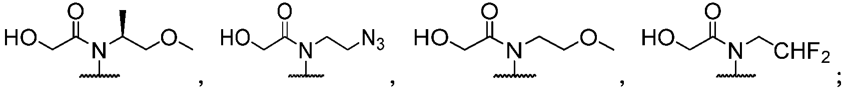
more preferably, R₃, R₄ and the nitrogen atom to which they connect collectively form

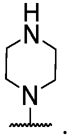

4. The compound or pharmaceutically acceptable salt, stereoisomer, or prodrug thereof according to any one of claims 1-3, wherein, R is selected from halogen, hydroxy, C₁-C₆ alkyl, C₁-C₆ alkoxy, C₃-C₆ cycloalkyl or azide;
preferably, R is selected from F, Cl, Br, I, hydroxy, methyl, methoxy, cyclopropyl or azide;
preferably, R is selected from F, hydroxy, methyl, methoxy, cyclopropyl or azide.

5. The camptothecin-7-ethylamine derivative or pharmaceutically acceptable salt, stereoisomer, or prodrug thereof according to any one of claims 1-4, wherein, the R₁, R₂ are each independently or collectively the following structure:

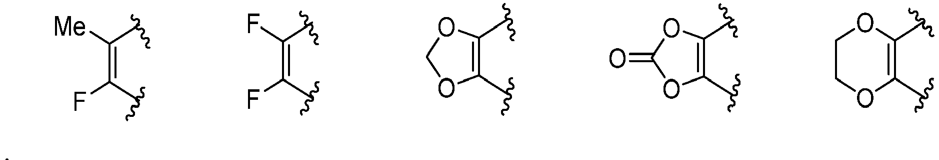

6. The compound or pharmaceutically acceptable salt, stereoisomer, or prodrug thereof according to any one of claims 1-5, wherein, the compound is selected from:
| | | |
|---|---|---|
| **1** | **2** | **3** |
| 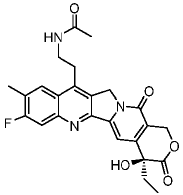 | 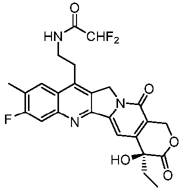 | 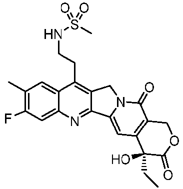 |
| **4** | **5** | **6** |
| 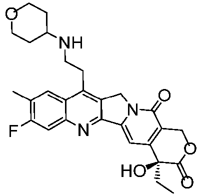 | 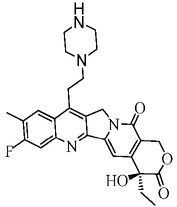 | 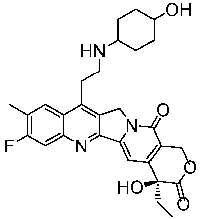 |
| **7** | **8** | **9** |
| 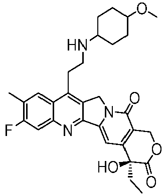 | 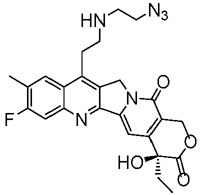 | 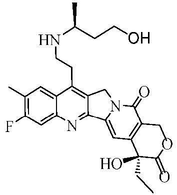 |
| **10** | **11** | **12** |
| 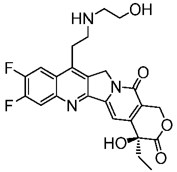 | 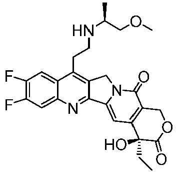 | 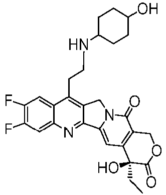 |
| **13** | **14** | **15** |
| 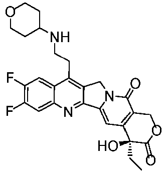 | 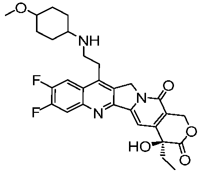 | 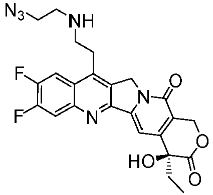 |
| **16** | **17** | **18** |
| 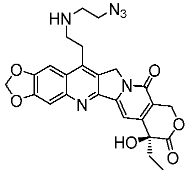 | 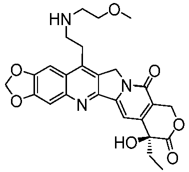 | 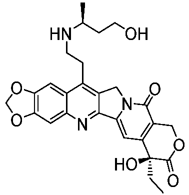 |
| **19** | **20** | **21** |
| 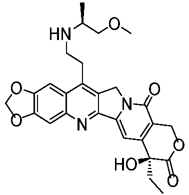 | 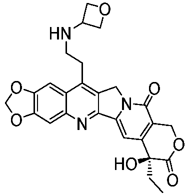 | 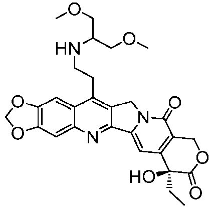 |
| **22** | **23** | **24** |
| 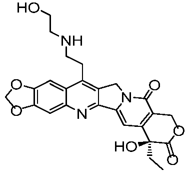 | 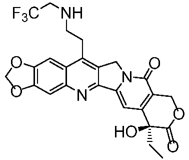 | 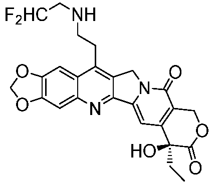 |
| **25** | **26** | **27** |
| 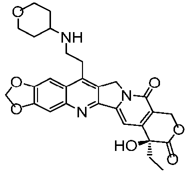 | 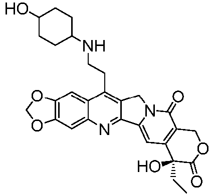 | 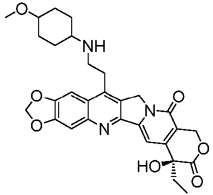 |
| **28** | **29** | **30** |
| 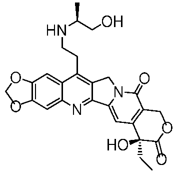 | 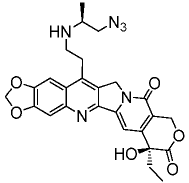 | 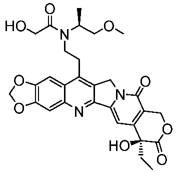 |
| **31** | **32** | **33** |
| 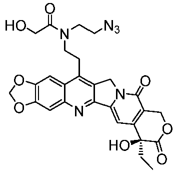 | 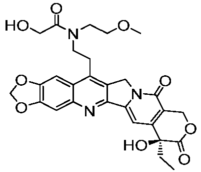 | 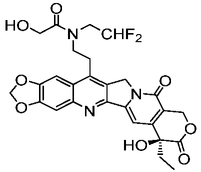 |
| **34** | **35** | **36** |
| 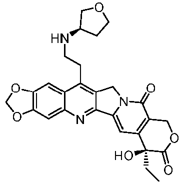 | 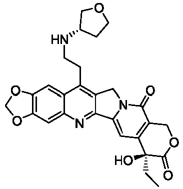 | 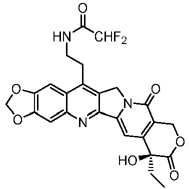 |
| **37** | **38** | **39** |
| 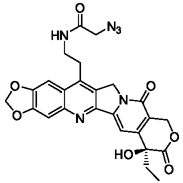 | 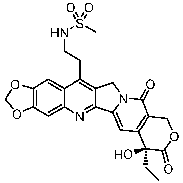 | 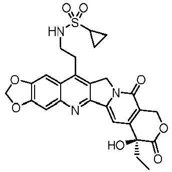 |
| **40** | **41** | **42** |
| 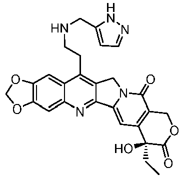 | 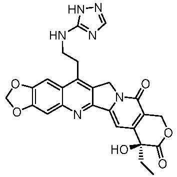 | 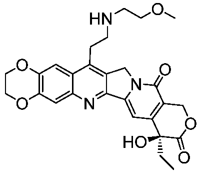 |
| **43** | **44** | **45** |
| 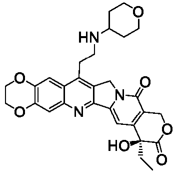 | 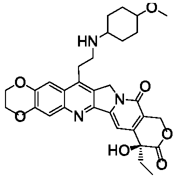 | 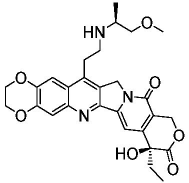 |

7. A method for preparing the compound according to any one of claims 1-6, **characterized in that** the preparation method comprises steps selected from any one of the following synthesis routes:
synthesis route 1, wherein, it comprises the following processes:
(1) 3,4-substituted aniline was reacted with the halopropionitrile to afford 3',4'-disubstituted-3-halo-6'-aminopropiophenone;
(2) 3',4'-disubstituted-3-halo-6'-aminopropiophenone was subjected to a substitution reaction with an amine to afford 3',4'-disubstituted-3-alkylamino-6'-aminopropiophenone;
(3) 3',4'-disubstituted-3-alkylamino-6'-aminopropiophenone was condensed with (S)-4-ethyl-4-hydroxy-7,8-dihydro-1H-pyranone-[3,4-f]indolizine-3,6,10(4H)-trione to afford the camptothecin-7-ethylamine derivatives; or,
synthesis route 2, wherein, it comprises the following processes:
(1) 3',4'-disubstituted-3-halopropiophenone was reacted with nitric acid to afford 6'-nitro-3',4'-disubstituted-3-halopropiophenone;
(2) 6'-nitro-3',4'-disubstituted-3-halopropiophenone was subjected to a substitution reaction with an amine to afford 6'-nitro-3',4'-disubstituted-3-alkylamino-propiophenone;
(3) 6'-nitro-3',4'-disubstituted-3-alkylamino-propiophenone was subjected to a reduction reaction to afford 6'-amino-3',4'-disubstituted-3-alkylamino-propiophenone;
(4) 3',4'-disubstituted-3-alkylamino-6'-aminopropiophenone was condensed with (S)-4-ethyl-4-hydroxy-7,8-dihydro-1H-pyranone-[3,4-f]indolizine-3,6,10(4H)-trione to afford the camptothecin-7-ethylamine derivative; or,
synthesis route 3, wherein, it comprises the following processes:
(1) 3',4'-disubstituted acetophenone was nitrated to afford 6'-nitro-3',4'-disubstituted acetophenone;
(2) 6'-nitro-3',4'-disubstituted acetophenone was condensed with formaldehyde and acidified to afford 6'-nitro-3',4'-disubstituted propenophenone;
(3) 6'-nitro-3',4'-disubstituted propenophenone was subjected to a Michael addition reaction with an amine to afford 6'-nitro-3',4'-disubstituted propiophenone-3-amine;
(4) 6'-nitro-3',4'-disubstituted propiophenone-3-amine was subjected to a reduction reaction to afford 6'-amino-3',4'-disubstituted propiophenone-3-amine;
(5) 6'-amino-3',4'-disubstituted propiophenone-3-amine was subjected to a condensation reaction with (S)-4-ethyl-4-hydroxy-7,8-dihydro-1H-pyranone-[3,4-f]indolizine-3,6,10(4H)-trione to afford the camptothecin-7-ethylamine derivatives; or,
synthesis route 4, wherein, it comprises the following processes:
(1) substituted 2-acetyl aniline was condensed with (S)-4-ethyl-4-hydroxy-7,8-dihydro-1H-pyranone-[3,4-f]indolizine-3,6,10(4H)-trione to afford 7-methylcamptothecin;
(2) 7-methylcamptothecin was subjected to a Mannich reaction with an amine in DMSO to afford the camptothecin-7-ethylamine derivative.

8. An antibody-drug conjugate, **characterized in that**, the compound or pharmaceutically acceptable salt, stereoisomer, or prodrug thereof according to any one of claims 1-6 is used as small molecule drug.

9. A pharmaceutical composition, **characterized by** comprising the compound or pharmaceutically acceptable salt, stereoisomer, or prodrug thereof according to any one of claims 1-6, or the antibody-drug conjugate according to claim 8 and a pharmaceutically acceptable excipient.

10. Use of the compound or pharmaceutically acceptable salt, stereoisomer, or prodrug thereof according to any one of claims 1-6, the antibody-drug conjugate according to claim 8, or the pharmaceutical composition according to claim 9 in the manufacture of a medicament for treating a cancer disease;
preferably, the cancer disease includes gastric cancer, esophageal cancer, cardia cancer, breast cancer, ovarian cancer, colon cancer, rectal cancer, primary liver cancer, acute and chronic granulocytic leukemia, choroidal epithelial cancer, lung cancer, bladder cancer, intestinal cancer and small cell lung cancer; more preferably, the cancer disease is esophageal cancer, breast cancer and gastric cancer.

11. A method for treating cancer comprising the step of administering to a patient in need thereof the compound or pharmaceutically acceptable salt, stereoisomer, or prodrug thereof according to any one of claims 1-6, the antibody-drug conjugate according to claim 8, or the pharmaceutical composition according to claim 9.
